# EUROPEAN PATENT SPECIFICATION

(11) **EP 3 119 438 B1**
(45) Date of publication and mention of the grant of the patent: **22.09.2021**
(21) Application number: 15710213.8
(22) Date of filing: 18.03.2015
(51) Int. Cl.: A61K 49/00, A61K 49/18, A61K 47/69, A61K 49/10, A61K 31/7048, A61P 31/00, A61P 35/00

(54) **MAGNETIC AND FLUORESCENT REVERSE NANOASSEMBLIES**
MAGNETISCHE UND FLUORESZIERENDE REVERSE-NANOANORDNUNGEN
NANOENSEMBLES MAGNÉTIQUES ET FLUORESCENTS À ARCHITECTURES INVERSES

(30) Priority: 18.03.2014 US 201414218368
(43) Date of publication of application: 25.01.2017
(73) Proprietor: Université de Nantes, 44000 Nantes (FR); Centre National de la Recherche Scientifique (CNRS), 75016 Paris (FR)
(72) Inventor: ISHOW, Eléna, 44000 Nantes (FR); FAUCON, Adrien, 44000 Nantes (FR)
(74) Representative: Icosa
(86) International application number: PCT/EP2015/055711
(87) International publication number: WO 2015/140223

(56) References cited:
- US-A1- 2013 030 282
- US-B1- 8 236 284
- JEROME FRESNAIS ET AL: "Electrostatic Co-assembly of Magnetic Nanoparticles and Fluorescent Nanospheres: A Versatile Approach Towards Bimodal Nanorods", SMALL, vol. 5, no. 22, 16 November 2009 (2009-11-16), pages 2533-2536, XP055192752, ISSN: 1613-6810, DOI: 10.1002/smll.200900703
- ADRIEN FAUCON ET AL: "Photoactive chelating organic nanospheres as central platforms of bimodal hybrid nanoparticles", JOURNAL OF MATERIALS CHEMISTRY C, vol. 1, no. 24, 1 January 2013 (2013-01-01), page 3879, XP055192551, ISSN: 2050-7526, DOI: 10.1039/c3tc30508b cited in the application
- RICCARDO DI CORATO ET AL: "Multifunctional Nanobeads Based on Quantum Dots and Magnetic Nanoparticles: Synthesis and Cancer Cell Targeting and Sorting", ACS NANO, vol. 5, no. 2, 22 February 2011 (2011-02-22), pages 1109-1121, XP055193271, ISSN: 1936-0851, DOI: 10.1021/nn102761t
- None

## Description

### FIELD OF INVENTION

The present invention relates to magnetic and fluorescent nanoassemblies having a reverse architecture as defined in the claims. Especially, the nanoassemblies of the invention comprise an organic fluorescent inner core and superparamagnetic nanoparticles at the surface thereof, leading to a core-shell architecture. The nanoassemblies of the invention further comprise a polymer adsorbed at its surface, said polymer being optionally functionalized. The invention further relates to a process for manufacturing the nanoassemblies of the invention. The invention is also directed to the use of the nanoassemblies of the invention, especially for multimodal imaging, *in vitro* and/or *in vivo* diagnostics through multimodal imaging, *ex vivo* sensing and/or extraction, and/or therapy.

### BACKGROUND OF INVENTION

Multimodal nanoparticles (NPs) advantageously possess properties such as a size similar to those of proteins or nucleic acids, large interactions due to their high surface area to volume ratio, complementary combination of active units within a single platform, high active material localization, structural diversity, and long circulation time in blood compared to small molecules.

The elaboration of bimodal nanoparticles, combining fluorescence and magnetism, has fostered great interest for their promising potential in dual bioimaging for diagnosis, remote drug vectorization, therapy by hyperthermia and selection of biological entities. While fluorescence detection is characterized by high sensitivity, addressing magnetic properties opens up the possibility of deep tissue imaging and remote mass transfer.

Most of the common systems that have been elaborated so far consist of iron oxide nanoparticles (magnetic), which are known for their relative innocuity, coated with organic or inorganic functional units (fluorescent). Alternative doped or core-shell structures have been elaborated with luminescent lanthanides or quantum dots, and yet these latter structures require prior encapsulation in silica or latex matrices to be protected from emission quenching by the surroundings. (Cheon and Lee, Acc. Chem. Res. 2008, 41, 1630-1640; Lee et al., Chem. Soc. Rev. 2012, 41, 2575-2589; Fan et al., ACS Nano 2012, 6, 1065-1073; Bigall et al., Nano Today, 2012, 7, 282-296).

For all these systems, deleterious electron transfer effects exerted by the magnetic nanoparticles on the luminophore emission have been noted, which is reinforced by the usually low payload of luminophores compared to that of iron oxide nanoparticles.

Moreover, these nanoparticles suffer from the following drawbacks:
- leakage or disassembling of the fluorescent molecules or the magnetic nanoparticles, especially in biological media, leading to a loss of combined fluorescence and magnetism, a decrease in the performances, and the appearance of noise signal;
- low payload of fluorescent and magnetic active units, compelling to use high doses;
- high photobleaching yield in the case of individual organic fluorescent molecules;
- high sensitivity of fluorescence towards the biological surroundings causing possible emission quenching or color shift;
- weak size discrimination leading to non-specific targeting of organs and/or malignant cells;
- low half-life in biological media, especially short circulation time in blood causing rapid clearance of materials following intravenous injection;
- difficulties to further functionalize the nanoparticle surface, whereas functionalization brings strong added value in terms of biological targeting and action.

Surprisingly, core-shell structures, based on an organic emissive platform (fluorescent) surrounded by magnetic nanoparticles have never been proposed to address these issues.

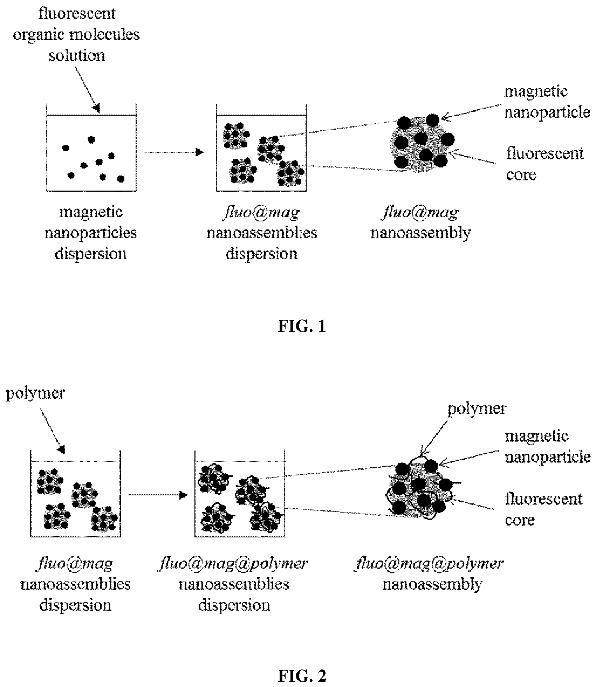

The present disclosure thus relates to nanoassemblies comprising a matrix-free organic fluorescent core surrounded by magnetic nanoparticles. The resulting reverse architecture of the nanoassemblies of the present disclosure resembles a "raspberry like" arrangement (Figure 1) (Faucon et al., J. Mater. Chem. C., 2013, 1, 3879-3886). In the following, such reverse architectures, which are not part of the invention, are referred to as *"fluo@mag"* nanoassemblies or "fluorescent and magnetic nanoassemblies".

The Applicant evidenced that such reverse architectures provide dense emissive core where the embedded fluorescent molecules become insensitive to the pH or the ionic strength of the media, and provide stable fluorescence signal under usual light observation conditions. Moreover, emission quenching from the magnetic nanoparticles through deleterious electron transfer is severely reduced due to effective separation of the magnetic nanoparticles from the fluorescent core. More than 70% of the core's fluorescence is retained after addition of the magnetic nanoparticles. Positioning magnetic nanoparticles on the periphery creates a charged shell structure which acts as a repelling electrostatic barrier to ions or hydrophobic molecules. As a result, fluorescent molecules are protected from possible dissolution in hydrophobic media or action by enzymes, which would result in the disassembling/release of intact or transformed individual fluorescent molecules. This in turn reinforces the fluorescent core cohesion through strong interactions of the fluorescent molecules, hence destabilization upon solvation is unlikely to occur. By contrast, the possible loss of magnetic nanoparticles would cause little effects in terms of spurious signal as the magnetic response of individual nanoparticles is smaller than that of the final bimodal assemblies.

The *fluo@mag* nanoassemblies of the present disclosure present many advantages as described above. However, their colloidal stability needs to be improved in biological medium. By "colloidal stability", it is herein referred to the ability of the nanoassemblies to remain dispersed in liquids without aggregating for long periods of time, preferably for at least several days (typically at least 3 days), more preferably at least 1 month, even more preferably at least 1 month in a physiological buffer or at least 6 months in purified water (such as Millipore water). There is thus a need for stabilization of the reverse nanoassemblies of the present disclosure in physiological aqueous conditions.

Moreover, according to one embodiment, the *fluo@mag* nanoassemblies may be obtained and stabilized under dilute acidic conditions, preferably using nitric acid (see the manufacturing process in the experimental part below). These acidic conditions are not optimized for biological purposes, especially for *in vivo* imaging. There is thus a need for stabilization of the nanoassemblies of the present disclosure in water and physiological conditions when they are obtained in acidic medium.

Furthermore, it would be interesting to functionalize the nanoassemblies of the present disclosure by bioactive molecules in order to widen the range of possible biological applications of the nanoassemblies. Care should be taken that functionalization of the nanoassemblies does not lead to the aggregation of nanoassemblies.

The Applicant evidenced that stabilization in physiological conditions and functionalization may be achieved by the addition of a polymeric protective shell around the nanoassembly of the present disclosure.

Especially, the Applicant showed that coating the surface of the nanoassembly of the present disclosure by adsorbing a polymer at its surface may achieve the above expected effects (Figure 2). In the following, such *fluo@mag* nanoassemblies coated with a polymer, which are according to the invention, are referred to as *fluo@mag@polymer* nanoassemblies or "polymer-coated fluorescent and magnetic nanoassemblies" or "nanoassembly systems".

The polymer used to modify the nanoassemblies of the present disclosure should meet the following specifications:
- Interactions between the polymer and the nanoassembly should provide overall stiff cohesion to the *fluo@mag@polymer* nanoassembly. For that purpose, electrostatic interactions with the positively charged surface of the nanoassemblies are preferred. Moreover, the polymer preferably comprises a multiplicity of sites interacting with the nanoassembly.
- The association constant between the polymer and the magnetic nanoparticles should be lower than the association constant between the fluorescent core and the magnetic nanoparticles. In other words, the polymer should have enough affinity for the magnetic nanoparticles present at the surface of the fluorescent core in order to coat the nanoassembly, while avoiding to take off part of the magnetic shell.
- The presence of the polymer should not induce the aggregation of the nanoassemblies both during manufacturing and during use in biological media.
- The polymer should be functionalizable, preferably the polymer should present a multiplicity of functionalizable patterns.
- The polymer should present biocompatibility.

The Applicant evidenced that negatively charged polyelectrolytes meet above specifications. According to a specific embodiment of the invention, the preferred polymer is poly(acrylic acid) (PAA), preferably the polymer is functionalized poly(acrylic acid).

Advantageously, the mean size of the *fluo@mag@polymer* does increase in a reasonable range compared to that of the uncoated *fluo@mag* nanoassembly, keeping the adequate dimensions for applications *in vivo.*

### SUMMARY

The present invention relates to a nanoassembly system comprising
- a nanoassembly comprising:
   ∘ an organic fluorescent inner core comprising fluorescent organic molecules, and
   ∘ magnetic nanoparticles contacting the surface of said fluorescent inner core; and
- at least one polymer adsorbed at the surface of the nanoassembly;
provided that the nanoassembly system does not comprise silica, functionalized silica, doped silica, grafted silica or a combination thereof,
wherein the fluorescent organic molecules are compounds of Formula (I), 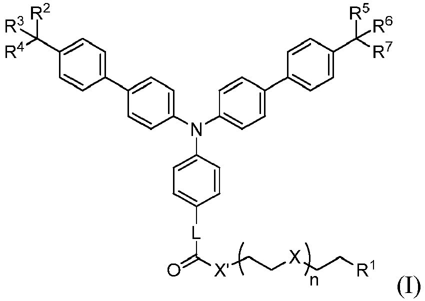 wherein:
**L** represents a spacer selected from (i), (ii) and (iii) 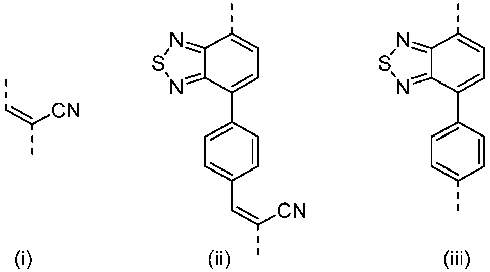
**X** represents O, CH₂ or NR⁸, wherein R⁸ represents H, alkyl or alkene;
**X'** represents O or NR⁹, wherein R⁹ represents H, alkyl or alkene;
**n** represents an integer selected from 1, 2, 3 and 4;
**R¹** represents -CO₂H or -P(O)(OH)₂;
**R²**, **R³, R⁴, R⁵, R⁶** and **R⁷** represent each independently an optionally functionalized group selected from alkyl and ester or poly(ethylene glycol), preferably **R²**, **R³, R⁴, R⁵, R⁶** and **R⁷** represent each a methyl group, and
wherein the magnetic nanoparticles are selected from the group consisting of γ-Fe₂O₃, Fe₃O₄, CoFe₂O₄, MnFe₂O₄, CuFe₂O₄, NiFe₂O₄.

According to one embodiment, in the nanoassembly system of the invention, the magnetic nanoparticles are superparamagnetic nanoparticles selected from the group consisting of γ-Fe₂O₃ and Fe₃O₄.

According to one embodiment, the nanoassembly system of the invention has a hydrodynamic diameter ranging from 20 to 700 nm.

According to one embodiment, in the nanoassembly system of the invention, the fluorescent inner core comprises a number of organic fluorescent molecule ranging from 1×10⁴ to 1×10⁷.

According to one embodiment, the nanoassembly system of the invention comprises a number of magnetic nanoparticles ranging from 1×10² to 1×10⁶.

According to one embodiment, in the nanoassembly system of the invention, the polymer is an ionic polymer, preferably a polyelectrolyte.

According to one embodiment, in the nanoassembly system of the invention, the polymer is of Formula (II), 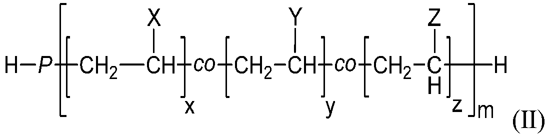 wherein,
**m** represents a positive integer ranging from 20 to 150;
**x, y** and **z** represent each independently a percentage of **m,** ranging from 0% to 100% of m, wherein x+y+z is equal to 100% of m;
**X** represents -COOH, alkyl, aryl, -CO-alkyl, poly(ethylene glycol), -poly(ethylene glycol)-OMe; **Y** represents -(C=O)-O-L¹-R⁸, -(C=O)-S-L¹-R⁸, - (C=O)-NH(-L¹-R⁸) or -(C=S)-NH(-L¹-R⁸) wherein
   **L¹** represents a spacer selected from alkyl, alkene, aryl, arylalkyl, poly(ethylene glycol) or poly(propylene glycol) linking groups having 1 to 150 chain atoms, wherein the linking group can be optionally interrupted or terminated by one or more -O-, -S-, -NR⁹-, -CO-, -NHCO-, -CONH- or a combination thereof, wherein R⁹ is H or alkyl;
   **R⁸** represents a reactive group selected from N₃, amino, alkylamino, COOH, amide, maleimide, alkene, alkyne, SH, OH, ester, activated ester, activated carboxylic acid, halo, nitro, nitrile, isonitriles, acrylamide, aldehyde, ketone, acetal, ketal, anhydride, glutaric anhydride, succinic anhydride, maleic anhydride, thiocyanate, isothiocyanate, isocyanate, hydrazide, hydrazine, hydrazone, ether, oxide, cyanate, diazo, diazonium, sulfide, disulfide, sulfoxide, sulfone, sulfonic acid, sulfinic acid, sulfate, sulfenic acid, amidine, imide, imidate, nitrone, hydroxylamine, oxime, hydroxamic acid, thiohydroxamic acid, allene, ortho ester, sulfite, enamine, ynamine, urea, pseudourea, semicarbazide, carbodiimide, carbamate, imine, acetylene, olefin, polyene, alkylacrylate, oxetane, ammonium, oxonium, phosphonium, sulfonium, positively charged or neutral metal complex;
**Z** represents -(C=O)-O-L²-R¹⁰, -(C=O)-S-L²-R¹⁰, -(C=O)-NH(-L²-R¹⁰) or - (C=S)-NH(-L²-R¹⁰) wherein
   **L²** represents a single bond or a spacer selected from alkyl, alkene, aryl, arylalkyl, poly(ethylene glycol) or poly(propylene glycol) linking groups having 1 to 150 chain atoms, wherein the linking group can be optionally interrupted or terminated by one or more -O-, -S-, -NR⁹-, -CO-, -NHCO-, -CONH- or a combination thereof, wherein R⁹ is H or alkyl; optionally additionally comprising a residue of a reactive group through which L² is bonded to R¹⁰;
   **R¹⁰** represents a bioactive group selected from amino acid, peptide, protein, antibody, affinity protein, enzyme, polysaccharide, dextran, benzylguanine, lipid, lipid assembly, fatty acid, nucleoside, nucleotide, oligonucleotide, hapten, aptamer, ligand, antibiotic, substrate, biotin, avidin, streptavidin, synthetic polymer, poly(ethylene glycol), poly(propylene glycol), polymeric microparticle, nanoparticle, fluorophore, chromophore, radioisotope, macrocyclic complexes of radioisotope, and combinations thereof.

According to one embodiment, in the nanoassembly system of the invention, the polymer is poly(acrylic acid).

The invention also relates to a process for manufacturing nanoassembly systems according to the invention, comprising injecting a solution of fluorescent organic molecules into a dispersion of magnetic nanoparticles under stirring, and a subsequent step of addition of the polymer.

According to one embodiment, the process of the invention further comprises a subsequent step of functionalization of the polymer by a bioactive group.

The invention further relates to a pharmaceutical composition comprising nanoassembly systems of the invention, in combination with at least one pharmaceutically acceptable vehicle.

The invention also relates to a medicament comprising nanoassembly systems according to the invention.

The invention also relates to the use of nanoassembly systems according to the invention, to extract and detect molecules and/or biological targets from physiological medium.

Moreover, the invention provides a kit comprising nanoassembly systems according to the invention.

The invention also relates to the use of the nanoassembly of the invention, for bimodal magnetic and fluorescent imaging. The invention also relates to the use of the nanoassembly of the invention, for cell sorting.

### DEFINITIONS

In the present invention, the following terms have the following meanings:
- **"nanoassembly"** refers to a nanometer-sized architecture wherein at least one dimension is lower than 700 nm, featuring the tight arrangement of nanoparticles. According to a preferred embodiment, the nanoassembly of the invention is of spherical shape.
- **"fluorescent"** refers to an emissive property following light excitation upon one- or two-photon excitation in the UV,- visible or near infrared (up to 900 nm) range.
- **"magnetic"** refers to the ability to respond to an applied external magnetic field upon alignment of magnetic dipoles along the direction of the magnetic field.
- **"superparamagnetic"** refers to the magnetic property encountered for nanoparticles smaller than a single magnetic monodomain. Such nanoparticles steadily orient upon applying an external magnetic field until a maximum value of the global magnetization, dubbed saturation magnetization, is reached. They relax when removing the magnetic field, with no magnetic hysteresis (no remanence) at room temperature. In the absence of an external magnetic field, superparamagnetic nanoparticles exhibit non-permanent magnetic moment due to thermal fluctuations of the dipole orientation (Neel relaxation) and nanoparticle position (Brownian relexation).
- **"hydrodynamic diameter"** refers to the diameter of nanoparticles in solution, as measured by dynamic light scattering, taking into account the extension of the first solvation shell around the nanoparticles.
- **"polyelectrolyte"** refers to a water-soluble macromolecule displaying a multiplicity of ionizable or ionic repeating units and able to electrostatically interact with the polarized surface of metal oxide materials, especially iron oxide nanoparticles.
- **"antibody"** as used herein includes monoclonal antibodies, polyclonal antibodies, multispecific antibodies (e.g., bispecific antibodies), and antibody fragments, so long as they exhibit the desired biological targeting.
- **"alkyl"** refers to any saturated linear, branched or cyclic hydrocarbon chain, with 1 to 50 carbon atoms, preferably 1 to 6 carbon atoms. Suitable alkyl groups include methyl, ethyl, propyl (n-propyl, i-propyl, n-butyl), butyl (i-butyl, s-butyl and t-butyl), pentyl and its isomers (e.g. n-pentyl, iso-pentyl), and hexyl and its isomers (e.g. n-hexyl, iso-hexyl). The alkyl group may be substituted or comprise heteroatoms.
- **"alkene"** refers to any linear or branched hydrocarbon chain having at least one carbon-carbon double bond, of 2 to 50 carbon atoms, and preferably 2 to 6 carbon atoms. The alkenyl group may be substituted. Examples of alkenyl groups are ethenyl, propen-2-yl, buten-2-yl, buten-3-yl, penten-2-yl and its isomers, hexen-2-yl and its isomers, pentadi-2,4-enyl and the like. The alkenyl group may be substituted by a saturated or unsaturated aryl group.
- **"alkyne"** refers to any linear or branched hydrocarbon chain having at least one carbon-carbon triple bond, of 2 to 50 carbon atoms, and preferably 2 to 6 carbon atoms. Non limiting examples of alkynyl groups are ethynyl, propyn-2-yl, butyn-2-yl, 3-butyn-3-yl, pentyn-2-yl and its isomers, hexyn-2-yl and its isomers-and the like.
- **"aryl"** refers to an aromatic hydrocarbon group having a single ring (i.e. phenyl) or multiple aromatic rings fused together (e.g. naphthyl) or linked covalently, typically containing 5 to 20 atoms; preferably 6 to 12, wherein at least one ring is aromatic. The aromatic ring may optionally be fused to one to two additional rings (either cycloalkyl, heterocyclyl or heteroaryl). Non-limiting examples of aryl comprise phenyl group, the biphenyl group, the 1-naphthyl group, the 2-naphthyl group, the tetrahydronaphthyl group, the indanyl group, the binaphthyl group, the fluorenyl group and the anthracenyl group.
- **"arylalkyl"** refers to an alkyl group substituted by an aryl group, such as for example the phenyl-methyl group.
- **"activated ester"** refers to an ester in which the alkoxy group is an electron-withdrawing group, for example N-hydroxysuccinimide ester, N-hydroxyglutarimide ester, N-hydroxybenzotriazole ester, maleimide ester or pentafluorophenyl ester.
- **"activated carboxylic acid"** refers for example to acid anhydride or acyl halide.
- **"spacer"** refers to a moiety bridging two parts of a molecule. Preferably the spacer is selected from alkyl, alkene, aryl, arylalkyl, poly(ethylene glycol) or poly(propylene glycol) linking groups having 1 to 150 chain atoms, wherein the linking group can be optionally interrupted or terminated by one or more -O-, -S-, -NR⁹-, -CO-, -NHCO-, -CONH- or a combination thereof, wherein R⁹ is H or alkyl.

In the case wherein the spacer is linked to a reactive group, the reactive group may be reacted with a substance reactive therewith, whereby the spacer becomes bonded to a bioactive group. In this case, the spacer typically contains a residue of the reactive group, such as for example the carbonyl group of an ester or a triazolyl group resulting from a click reaction between an azide and an alkyne. Such residue are known by one skilled in the art. By "triazolyl group" it is referred to the following moiety:

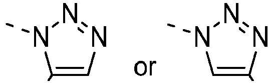

For example, binding between the reactive groups present on the spacer and on the bioactive group may lead to the following bonds:

| **reactive group I** | **reactive group II** | **resulting bond** |
|---|---|---|
| R-NH₂ | R'-COOH | R-NH(C=O)-R' |
| R-SH | R'-SH | R-SS-R' |
| R-OH | R'-(epoxyde group) | R-OCH₂CH(OH)-R' |
| R-NH₂ | R'-(epoxyde group) | R-NHCH₂CH(OH)-R' |
| R-SH | R'-(epoxyde group) | R-SCH₂CH(OH)-R' |
| R-NH₂ | R'-CHO | R-N=CH-R' |
| R-NH₂ | R'-NCO | R-NH(C=O)NH-R |
| R-NH₂ | R'-NCS | R-NH(C=S)NH-R' |
| R-SH | R' -(C=O)CH₃ | R-(C=O)CH₂S-R' |
| R-SH | R'-O(C=O)X | R-S(C=O)O-R' |
| R-CH=CH₂ | R'-SH | R-CH₂CH₂S-R' |
| R-OH | R'-NCO | R-O(C=O)NH-R' |
| R-SH | R'-(C=O)CH₂X | R-SCH₂(C=O)-R' |
| R-NH₂ | R'-(C=O)N₃ | R-NH(C=O)-R' |
| R-COOH | R'-COOH | R-(C=O)O(C=O)-R' |
| R-SH | R'-X | R-S-R' |
| R-NH₂ | R-CH₂C(NH²⁺)OCH₃ | R-NHC(NH²⁺)CH₂-R' |
| R-NHNH₂ | R'-CHO | R-NHNH=CH-R' |

wherein X represents an halogen.

### DETAILED DESCRIPTION

### A. Nanoassemblies

### A.1. Fluo@mag nanoassemblies: fluorescent core and magnetic nanoparticles (not according to the invention)

The present disclosure relates to fluorescent and magnetic nanoassemblies (*fluo@mag* nanoassemblies). Especially, the nanoassembly of the present disclosure comprises a fluorescent core, preferably an organic fluorescent core, surrounded by magnetic nanoparticles contacting the surface of the fluorescent core, preferably superparamagnetic nanoparticles.

According to one disclosure, the nanoassembly of the present disclosure comprises
- a fluorescent core comprising fluorescent organic molecules, and
- magnetic nanoparticles contacting the surface of said fluorescent core.

According to one disclosure, the nanoassembly of the present disclosure comprises
- a fluorescent core comprising fluorescent organic molecules, and
- superparamagnetic nanoparticles contacting the surface of said fluorescent core.

Figure 1 illustrates the reverse architecture of the *fluo@mag* nanoassembly, which is not part of the invention. It is referred to "reverse" architecture, by contrast with the traditional magnetic and fluorescent nanoassemblies of the prior art in which the core is magnetic and coated by fluorescent units.

In the nanoassembly of the present disclosure, the fluorescent core and the magnetic nanoparticles are not linked by covalent bonds.

### Fluorescent core

According to one embodiment, the fluorescent core of the nanoassembly is an organic fluorescent core. Preferably, the fluorescent core is "matrix-free", *i.e.* does not comprise a matrix, wherein the matrix may be for example silica, oil droplets and vesicles, polymer (such as for example dextran, polyethyleneimine, polystyrene), liquid crystal micelles, liposomes, polymersomes, nanocapsules, gelatin nanoparticles, nanolatexes. In other words, the fluorescent core of the invention is not a matrix wherein fluorescent organic molecules are encapsulated or embedded.

According to one embodiment, the fluorescent inner core of the nanoassembly of the present disclosure is not silica, functionalized silica, doped silica, grafted silica or a combination thereof.

According to one embodiment, the fluorescent inner core of the nanoassembly of the present disclosure is not a quantum dot.

According to one embodiment, the fluorescent inner core of the nanoassembly of the present disclosure does not comprises a polymeric matrix, preferably does not comprise a cross-linked polymeric matrix.

According to one embodiment, the fluorescent inner core of the nanoassembly of the present disclosure does not comprise self-assembled non-fluorescent molecules or non-fluorescent macromolecules forming micelles or polymersomes respectively.

According to one embodiment, the fluorescent inner core of the nanoassembly of the present disclosure does not comprise self-assembled fluorescent macromolecules, preferably does not comprise self-assembled semiconducting polymer chains, forming polymer-dots (also called P-dots).

According to one embodiment, the fluorescent core of the nanoassembly of the present disclosure comprises fluorescent organic molecules, preferably small fluorescent organic molecules. According to one embodiment, the fluorescent core of the nanoassembly of the present disclosure comprises self-assembled fluorescent organic molecules. According to another embodiment, the fluorescent core of the nanoassembly of the present disclosure consists of fluorescent organic molecules, preferably small fluorescent organic molecules.

Preferably, the fluorescent core is of spherical shape.

The fluorescent core presents the advantage to have a high density of fluorescent molecules and weak emission quenching in the solid-state.

The fluorescent core may be obtained by a simple manufacturing process. According to one embodiment, the fluorescent core may be obtained following a one-step nucleation process.

According to one embodiment, the fluorescent organic molecules of the fluorescent core are those described in Faucon el al. J. Mater. Chem. C, 2013, 1 (24), 3879-3886.

According to one embodiment, the fluorescent organic molecules of the fluorescent core are compounds of Formula I: 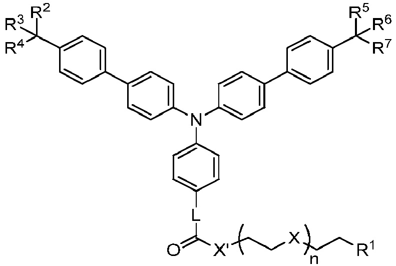 wherein:
**L** represents a spacer selected from (i), (ii) and (iii) 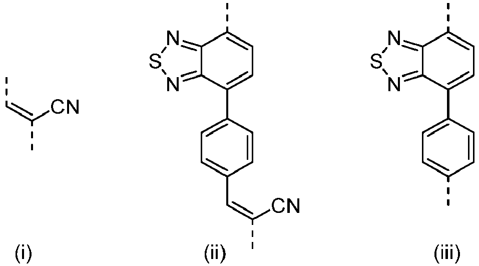
**X** represents O, CH₂ or NR⁸, wherein R⁸ represents H, alkyl or alkene;
**X'** represents O or NR⁹, wherein R⁹ represents H, alkyl or alkene;
**n** represents an integer selected from 1, 2, 3 and 4;
**R¹** represents -CO₂H or -P(O)(OH)₂;
**R²**, **R³, R⁴, R⁵, R⁶** and **R⁷** represent each independently an optionally functionalized group selected from alkyl and ester or poly(ethylene glycol), preferably **R²**, **R³, R⁴, R⁵, R⁶** and **R⁷** represent each a methyl group.

According to one embodiment, the fluorescent organic molecules of the fluorescent core of formula I are compounds of Formula I': 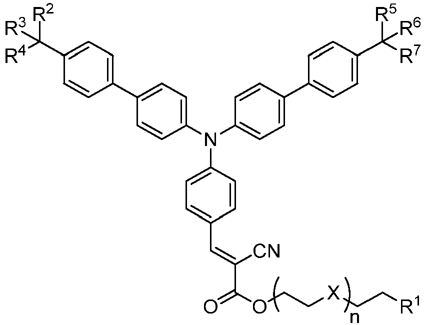 wherein:
**X** represents O or CH₂;
**n** represents an integer selected from 1, 2, 3 and 4;
**R¹** represents -CO₂H or -P(O)(OH)₂;
**R²**, **R³, R⁴, R⁵, R⁶** and **R⁷** represent each independently an optionally functionalized group selected from alkyl and ester or poly(ethylene glycol), preferably **R²**, **R³, R⁴, R⁵, R⁶** and **R⁷** represent each a methyl group.

According to a specific embodiment of the invention, the fluorescent organic molecules of the fluorescent core are of Formula (Ia) or (Ib):

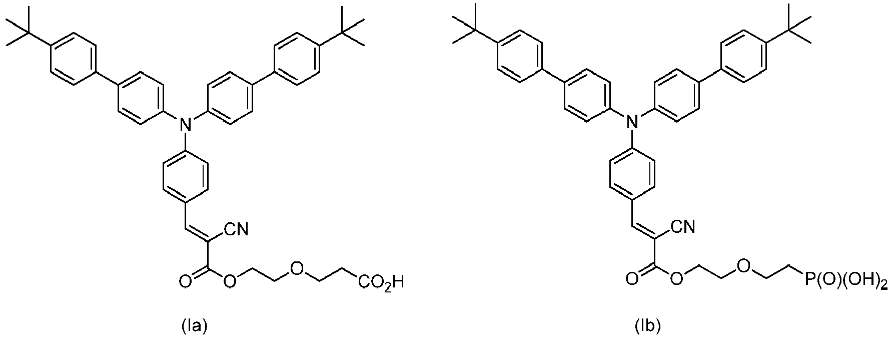

In one embodiment, the absorption wavelength of the fluorescent core is ranging from 200 to 800 nm, preferably from 300 to 650 nm, more preferably from 400 to 500 nm.

In one embodiment, the emission wavelength of the fluorescent core is ranging from 300 to 1000 nm, preferably from 400 to 800 nm, more preferably from 500 to 700 nm, more preferably from 550 to 700 nm.

According to one embodiment, the fluorescent core comprises a number of organic fluorescent molecules ranging from 1×10⁴ to 1×10⁷, preferably from 1×10⁴ to 1×10⁶, more preferably from 1×10⁵ to 1×10⁶.

In one embodiment, the fluorescent organic molecule comprises an ionizable group, preferably an anionic group, which is able to complex magnetic nanoparticles.

According to one embodiment, the fluorescent core further comprises active agents such as for example therapeutically active agents. According to another embodiment, the fluorescent core of the nanoassembly of the present disclosure consists of fluorescent organic molecules and active agents, such as for example therapeutically active agents.

### Magnetic nanoparticles

According to a preferred embodiment, the magnetic nanoparticles of the nanoassemblies of the present disclosure are superparamagnetic nanoparticles. Superparamagnetic particles become magnetized up to their saturation magnetization by applying an external magnetic field, while they do not possess any residual magnetization at room temperature when the magnetic field is removed.

According to one embodiment, the magnetic nanoparticles are selected from the group consisting of γ-Fe₂O₃, Fe₃O₄, CoFe₂O₄, MnFe₂O₄, CuFe₂O₄, NiFe₂O₄. Preferably, magnetic nanoparticles are nanoparticles of maghemite iron oxide γ-Fe₂O₃ or magnetite Fe₃O₄. According to one embodiment, the magnetic nanoparticles are superparamagnetic nanoparticles selected from the group consisting of γ-Fe₂O₃ and Fe₃O₄. According to a specific embodiment, magnetic nanoparticles are nanoparticles of maghemite iron oxide γ-Fe₂O₃. According to another specific embodiment, magnetic nanoparticles are nanoparticles of magnetite Fe₃O₄.

According to one embodiment, the nanoassembly of the present disclosure comprises a number of magnetic nanoparticles ranging from 1×10² to 1×10⁶, preferably from 1×10³ to 1×10⁵, more preferably from 0.5×10⁴ to 5×10⁴.

Advantageously, the magnetic nanoparticles are superparamagnetic and possess enhanced relaxivity properties upon tight association at the surface of the fluorescent core compared to those of free nanoparticles.

According to one embodiment, the magnetic nanoparticles present saturation magnetization Ms values ranging from 20 to 100 emu g⁻¹, preferably from 15 to 80 emu g⁻¹, preferably from 20 to 55 emu g⁻¹

According to one embodiment, compared to free nanoparticles, the magnetic nanoparticles within the nanoassembly of the present disclosure present r₂/r₁ ratio larger than 2, preferably larger than 4, more preferably larger than 6 at clinical MRI frequencies.

Advantageously, the magnetic nanoparticles present in the nanoassemblies of the present disclosure exert cooperative effects, leading to an important improvement of the MRI (magnetic resonance imaging) contrast. According to one embodiment, the magnetic nanoparticles present in the nanoassemblies of the present disclosure exert additive effects, leading to an important improvement of the MRI contrast. Therefore, the nanoassemblies of the present disclosure enable MRI imaging even at low iron concentrations.

### Nanoassembly properties

In a preferred embodiment, the fluorescent core of the nanoassembly of the present disclosure comprises fluorescent organic molecules of Formula (Ia) and the magnetic nanoparticles are nanoparticles of γ-Fe₂O₃ or Fe₃O₄. In a specific embodiment, the fluorescent core comprises fluorescent organic molecules of Formula (Ia) and the magnetic nanoparticles are nanoparticles of γ-Fe₂O₃. In another specific embodiment, the fluorescent core comprises fluorescent organic molecules of Formula (Ia) and the magnetic nanoparticles are nanoparticles of Fe₃O₄.

In a preferred embodiment, the fluorescent core of the nanoassembly of the present disclosure comprises fluorescent organic molecules of Formula (Ib) and the magnetic nanoparticles are nanoparticles of γ-Fe₂O₃ or Fe₃O₄. In a specific embodiment, the fluorescent core comprises fluorescent organic molecules of Formula (Ib) and the magnetic nanoparticles are nanoparticles of γ-Fe₂O₃. In another specific embodiment, the fluorescent core comprises fluorescent organic molecules of Formula (Ib) and the magnetic nanoparticles are nanoparticles of Fe₃O₄.

Advantageously, the fluorescent organic molecules and the magnetic nanoparticles comprised in the nanoassembly of the present disclosure are such that the emission wavelength of the fluorescent core is red-shifted compared to the absorption wavelength of the magnetic nanoparticles in order to avoid emission quenching upon inner-filter effect.

According to one embodiment, the fluorescent organic molecules have a Stokes shift higher than 4500 cm⁻¹.

In one embodiment of the invention, the hydrodynamic diameter of the nanoassembly is ranging from 20 to 800 nm, preferably from 50 to 500 nm, more preferably from 80 to 200 nm. According to one embodiment, the nanoassembly of the present disclosure has a hydrodynamic diameter ranging from 20 to 700 nm.

In a specific embodiment, when the nanoassembly comprises fluorescent organic molecules of Formula (Ia) and/or (Ib) and γ-Fe₂O₃ magnetic nanoparticles, the hydrodynamic diameter is preferably ranging from 80 to 200 nm.

In one embodiment, the nanoassemblies of the present disclosure are stored in acidic media. In this case, anionic counterions enable the stabilization of the nanoassemblies through electrostatic repulsion, which avoids aggregation of the nanoassemblies.

In another embodiment, the nanoassemblies of the present disclosure are stored in basic media. In this case, cationic counterions enable the stabilization of the nanoassemblies through electrostatic repulsion, which avoids aggregation of the nanoassemblies.

### A.2. Fluo@mag@polymer nanoassembly systems: fluorescent core, magnetic nanoparticles and polymer (according to the invention)

The present invention relates to nanoassembly systems comprising a magnetic and fluorescent nanoassembly, further comprising a coating of polymers *(fluo@mag@polymer* nanoassembly) as defined in the claims. Especially, the nanoassembly systems of the invention comprise an organic fluorescent core, partially or totally surrounded by magnetic nanoparticles contacting the surface of the fluorescent core, preferably superparamagnetic nanoparticles, and further continuously or discontinuously coated by polymers. The polymer is adsorbed at the surface of the nanoassembly. The polymer is not linked by covalent bonds to the nanoassembly.

Therefore, according to one embodiment, the nanoassembly of the present disclosure further comprises at least one polymer. According to one embodiment, the polymer is an ionic polymer, preferably a polyelectrolyte.

The constituents of the *fluo@mag@polymer* nanoassembly are defined in the claims.

Advantageously, the polymer replaces the anionic counterions used to stabilize the *fluo@mag* nanoassembly in acidic medium and thus allows for the stabilization of the nanoassembly in neutral medium. Advantageously, since the *fluo@mag@polymer* nanoassembly is stable in neutral medium, it can be used in biological medium.

In one embodiment, the *fluo@mag@polymer* nanoassembly of the invention is stable at a pH ranging from 3 to 12.

In one embodiment, the *fluo@mag@polymer* nanoassembly of the invention is stable at an ionic strength ranging from 0 to 1 mol/L of NaCl, preferably from 0.05 to 0.3 mol/L of NaCl.

According to one embodiment, the nanoassembly of the invention comprises:
- a fluorescent core, comprising fluorescent organic molecules;
- magnetic nanoparticles contacting the surface of the fluorescent core; and
- at least one polymer.

According to one preferred embodiment, the nanoassembly system of the invention comprises:
- a nanoassembly comprising:
   ∘ an organic fluorescent inner core, comprising fluorescent organic molecules; and
   ∘ magnetic nanoparticles contacting the surface of the fluorescent inner core;
   and
- at least one polymer adsorbed at the surface of the nanoassembly.

According to one embodiment, the nanoassembly system of the invention does not comprise silica, functionalized silica, doped silica, grafted silica or a combination thereof.

The polymer is preferably contacting the surface of the nanoassembly comprising a fluorescent core having at its surface magnetic nanoparticles. Figure 2 illustrates the architecture of the *fluo@mag@polymer* nanoassembly of the invention.

According to one embodiment, the *fluo@mag* nanoassemblies of the present disclosure further comprise at least one polymer at their surface, leading to *fluo@mag@polymer* nanoassemblies.

The *fluo@mag@polymer* nanoassemblies of the invention are advantageously hydrophilic, colloidally stable in acidic and alkaline media, photostable under irradiation, do not possess apparent cytotoxicity, present magnetic and fluorescence properties close or superior to those of the constitutive fluorescent core and magnetic nanoparticles, and do not deaggregate into their constitutive fluorescent and magnetic nanoparticles or molecules.

It was evidenced that the addition of the polymer protective shell leads to a minimal (namely about 40 nm) increase of the hydrodynamic diameter of the assembly only. In one embodiment, the *fluo@mag@polymer* nanoassembly has a hydrodynamic diameter ranging from 50 to 800 nm, preferably from 70 to 600 nm, more preferably from 90 to 300 nm. In one embodiment, the *fluo@mag@polymer* nanoassembly has a hydrodynamic diameter ranging from 150 to 400 nm.

The *fluo@mag@polymer* nanoassemblies may be stored after lyophilization. Re-dispersion after lyophilization may easily be achieved. According to one embodiment, re-dispersion after lyophilization is performed in a solvent selected from water, buffer, alcoholic solvents.

The *fluo@mag@polymer* nanoassemblies of the invention present the advantage not to aggregate when placed in a solvent. According to one embodiment, the solvent may be an alcoholic solvent, preferably ethanol or methanol.

The *fluo@mag@polymer* nanoassemblies of the invention present the advantage to be internalized in cells while not penetrating into the nucleus.

### Polymer

In one embodiment, the polymer used to continuously or discontinuously coat the nanoassembly of the invention is an ionic polymer, preferably a polyelectrolyte.

According to one embodiment, the polymer comprises anionic functions able to interact with the magnetic particles of the nanoassemblies of the invention.

According to one embodiment, the polymer is not cross-linked.

According to one embodiment, the polymer is a "functionalizable polymer" and/or a "functionalized polymer".

According to one embodiment, the polymer is a "functionalizable polymer", i.e. the polymer comprises at least one reactive group. By "reactive group", it is herein referred to a group capable of reacting with another chemical group to form a covalent bond and/or an electrostatic bond, i.e. is covalently reactive and/or ionically reactive under suitable reaction conditions, and generally represents a point of attachment for another substance. The reactive group is a moiety on the polymer that is capable of chemically reacting with a functional group on a different compound to form a covalent linkage and/or an electrostatic linkage. Reactive groups generally include nucleophiles, electrophiles, dienophiles, dipolarophiles, cations and photoactivable groups.

When a reactive group is present on the polymer used in the nanoassembly of the invention, it is possible to functionalize the nanoassembly.

According to one embodiment, the polymer comprises at least one reactive group selected from the group comprising N₃, amino, alkylamino, COOH, amide, maleimide, alkene, alkyne, SH, OH, ester, activated ester, activated carboxylic acid, halo, nitro, nitrile, isonitriles, acrylamide, aldehyde, ketone, acetal, ketal, anhydride, glutaric anhydride, succinic anhydride, maleic anhydride, thiocyanate, isothiocyanate, isocyanate, hydrazide, hydrazine, hydrazone, ether, oxide, cyanate, diazo, diazonium, sulfide, disulfide, sulfoxide, sulfone, sulfonic acid, sulfinic acid, sulfate, sulfenic acid, amidine, imide, imidate, nitrone, hydroxylamine, oxime, hydroxamic acid, thiohydroxamic acid, allene, ortho ester, sulfite, enamine, ynamine, urea, pseudourea, semicarbazide, carbodiimide, carbamate, imine, acetylene, olefin, polyene, alkylacrylate, oxetane, ammonium, oxonium, phosphonium, sulfonium, positively charged or neutral metal complex.

According to one embodiment, the polymer comprises at least one reactive group selected from the group comprising N₃, amino, alkylamino, COOH, amide, maleimide, alkyne, SH, OH, ester, activated ester, activated carboxylic acid, halo, nitro, nitrile, isonitriles, acrylamide, aldehyde, ketone, acetals, ketals, anhydride, glutaric anhydride, succinic anhydride, maleic anhydride, thiocyanate, isothiocyanate, isocyanate, hydrazide, hydrazines, hydrazones, ethers, oxides, cyanates, diazo, diazonium, sulfides, disulfides, sulfoxides, sulfones, sulfonic acids, sulfinic acids, sulfates, sulfenic acids, amidines, imides, imidates, nitrones, hydroxylamines, oximes, hydroxamic acids, thiohydroxamic acids, allenes, ortho esters, sulfites, enamines, ynamines, ureas, pseudoureas, semicarbazides, carbodiimides, carbamates, imines, acetylene, olefins, polyenes, alkylacrylates, oxetane, ammoniums, oxoniums, phosphoniums, sulfoniums, positively charged metal complexes.

According to one embodiment, the polymer comprises at least one reactive group being maleimide. According to one embodiment, the polymer comprises at least one reactive group being isothiocyanate. According to one embodiment, the polymer comprises at least one reactive group being an activated ester, preferably N-hydroxysuccinimide ester.

In one embodiment, the polymer comprises at least one lateral chain substituted by at least one reactive group.

According to another embodiment, the polymer is a "functionalized polymer", i.e. the polymer comprises at least one bioactive group. The bioactive group may enable to target the nanoassembly coated with the polymer by the recognition of biological targets. The bioactive group may also confer biological activity to the nanoassembly coated with the polymer.

According to one embodiment, the following bioactive groups may enable to recognize the corresponding biological targets:

| **bioactive group** | **biological target** |
|---|---|
| biotin | avidin, streptavidin, deglycolysated streptavidins, deglycolysated avidins |
| human immunoglobulines (IgG) | antigen |
| immunoglobulines (IgE) | antigen |
| oligonucleotides | DNA strands |
| tyrosine kinase inhibitors | antibodies |
| herceptin | epidermal growth factor receptors HER2 |
| aptamers | proteins, lysozymes |
| RGD peptides | αᵥβ₃ integrins |
| epidermal growth factor (EGF) inhibitors | epidermal growth factor receptors (EGFR) |
| β-lactams | transpeptidases |
| glycopeptides | transglycosylases |
| human immunodeficiency virus (HIV)1 transactivator of transcription (TAT) protein | cell nucleus |

According to one embodiment, the bioactive group is selected from amino acid, peptide, protein, antibody, enzyme, polysaccharide, dextran, benzylguanine, lipid, lipid assembly, fatty acid, nucleoside, nucleotide, oligonucleotide, hapten, aptamer, ligand, substrate, biotin, avidin, synthetic polymer, poly(ethylene glycol), poly(propylene glycol), polymeric microparticle, nanoparticle, fluorophore, chromophore, radioisotope, macrocyclic complexes of radioisotope, and combinations thereof.

According to one embodiment, the bioactive group is selected from amino acid, peptide (such as for example RGD peptides), protein (such as for example (HIV)1 TAT protein), antibody (such as for example human immunoglobulines IgG, immunoglobulines IgE, herceptine), affinity protein, enzyme, polysaccharide, dextran, benzylguanine, lipid, lipid assembly, fatty acid, nucleoside, nucleotide, oligonucleotide, hapten, aptamer, ligand (such as for example tyrosine kinase inhibitors or epidermal growth factor inhibitors), antibiotic (such as for example β -lactams or glycopeptides) substrate, biotin, avidin, streptavidin, synthetic polymer, poly(ethylene glycol), poly(propylene glycol), polymeric microparticle, nanoparticle, fluorophore, chromophore, radioisotope, macrocyclic complexes of radioisotope, and combinations thereof.

According to one embodiment, the bioactive group is selected from biotin, human immunoglobulines (IgG), immunoglobulines (IgE), oligonucleotides, tyrosine kinase inhibitors, herceptin, aptamers, RGD peptides, epidermal growth factor inhibitors, β - lactams, glycopeptides and (HIV)1 TAT proteins.

According to one embodiment, the bioactive group is an affinity protein. The term "affinity protein" refers to artificial proteins having the ability to selectively bind complementary proteins or antigens, i.e. being antibody mimetics. According to a specific embodiment, affinity proteins are of about 7 kDa. Preferably affinity proteins do not comprise disulfide bonds. According to a specific embodiment, affinity proteins are able to resist to high temperatures, high pressures and strongly acidic conditions.

The bioactive group may also impart the polymer-coated nanoassembly with furtivity. In this way, clearance of the nanoassembly from the bloodstream by the mononuclear phagocyte system (reticuloendothelial system (RES)) is impaired and longer circulation times in the body are obtained. In this case, the bioactive group preferably comprises or consists in a PEG moiety. According to a specific embodiment, the polymer is a PAA-PEG polymer.

According to one embodiment, the bioactive group is directly present on the polymer. Alternatively, the bioactive group is introduced on the polymer through reaction with a reactive group. Especially, the bioactive group may be introduced on the polymer by peptide coupling or by click chemistry. For example, an amine-derivated biotin group may be introduced on a PAA polymer by peptide coupling with the carboxylic units of the PAA.

Optionally, a spacer may be present between the polymer and the bioactive group.

In one embodiment, the bioactive group is present on a lateral chain of the polymer. According to another embodiment, the bioactive group is present at one extremity of the polymer or at both extremities.

Advantageously, the functionalization of the polymer by bioactive groups occurs without any loss of the biological properties of said bioactive groups.

In one embodiment, the polymer, and especially the "functionalizable polymer", has a molecular weight ranging from 1 to 10 kDa, preferably from 1 to 5 kDa. The "functionalized polymer" may have a molecular weight greater than 10 kDa, depending on the nature of the bioactive group used for functionalization.

Commonly used nomenclature for a copolymer comprising a total of m monomers, x% of said monomers being monomer A, y% of said monomers being monomer B and z% of said monomers being monomer C is: *P*[Aₓ-*co*-B_{y}-*co-*C_{z}]m. When the extremities of the copolymer are specific, they may be indicated on either side of *P*[Aₓ-_{co}-B_{y}-_{co}-C_{z}]m, namely under the form: R¹-*P*[Aₓ-*co*-B_{y}-*co*-C_{z}]m-R². This nomenclature is used hereafter.

In a specific embodiment, the polymer is poly(acrylic acid) or a derivative thereof of formula II: 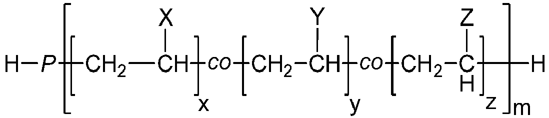 wherein,
**m** represents a positive integer ranging from 20 to150;
**x, y** and **z** represent each independently a percentage of **m,** ranging from 0% to 100% of m, wherein x+y+z is equal to 100% of m;
**X** represents -COOH, alkyl, aryl, -CO-alkyl, poly(ethylene glycol), -poly(ethylene glycol)-OMe;**Y** represents -(C=O)-O-L¹-R⁸, -(C=O)-S-L¹-R⁸, -(C=O)-NH(-L¹-R⁸) or -(C=S)-NH(-L¹-R⁸) wherein
   **L¹** represents a spacer selected from alkyl, alkene, aryl, arylalkyl, poly(ethylene glycol) or poly(propylene glycol) linking groups having 1 to 150 chain atoms, wherein the linking group can be optionally interrupted or terminated by one or more -O-, -S-, -NR⁹-, -CO-, - NHCO-, -CONH- or a combination thereof, wherein R⁹ is H or alkyl; **R⁸** represents a reactive group selected from N₃, amino, alkylamino, COOH, amide, maleimide, alkene, alkyne, SH, OH, ester, activated ester, activated carboxylic acid, halo, nitro, nitrile, isonitriles, acrylamide, aldehyde, ketone, acetal, ketal, anhydride, glutaric anhydride, succinic anhydride, maleic anhydride, thiocyanate, isothiocyanate, isocyanate, hydrazide, hydrazine, hydrazone, ether, oxide, cyanate, diazo, diazonium, sulfide, disulfide, sulfoxide, sulfone, sulfonic acid, sulfinic acid, sulfate, sulfenic acid, amidine, imide, imidate, nitrone, hydroxylamine, oxime, hydroxamic acid, thiohydroxamic acid, allene, ortho ester, sulfite, enamine, ynamine, urea, pseudourea, semicarbazide, carbodiimide, carbamate, imine, acetylene, olefin, polyene, alkylacrylate, oxetane, ammonium, oxonium, phosphonium, sulfonium, positively charged or neutral metal complex;
**Z** represents -(C=O)-O-L²-R¹⁰, -(C=O)-S-L²-R¹⁰, -(C=O)-NH(-L²-R¹⁰) or - (C=S)-NH(-L²-R¹⁰) wherein
   **L²** represents a single bond or a spacer selected from alkyl, alkene, aryl, arylalkyl, poly(ethylene glycol) or poly(propylene glycol) linking groups having 1 to 150 chain atoms, wherein the linking group can be optionally interrupted or terminated by one or more -O-, -S-, -NR⁹-, - CO-, -NHCO-, -CONH- or a combination thereof, wherein R⁹ is H or alkyl; optionally additionally comprising a residue of a reactive group through which L² is bonded to R¹⁰;
   **R¹⁰** represents a bioactive group selected from amino acid, peptide (such as for example RGD peptides), protein (such as for example (HIV)1 TAT protein), antibody (such as for example human immunoglobulines IgG, immunoglobulines IgE, herceptine), affinity protein, enzyme, polysaccharide, dextran, benzylguanine, lipid, lipid assembly, fatty acid, nucleoside, nucleotide, oligonucleotide, hapten, aptamer, ligand (such as for example tyrosine kinase inhibitors or epidermal growth factor inhibitors), antibiotic (such as for example β - lactams or glycopeptides) substrate, biotin, avidin, streptavidin, synthetic polymer, poly(ethylene glycol), poly(propylene glycol), polymeric microparticle, nanoparticle, fluorophore, chromophore, radioisotope, macrocyclic complexes of radioisotope, and combinations thereof.

In a specific embodiment, the polymer is poly(acrylic acid) or a derivative thereof of formula II': 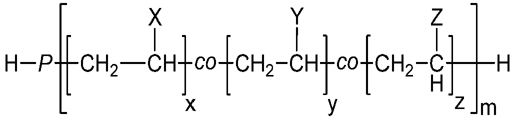 wherein,
**m** represents a positive integer ranging from 20 to150;
**x, y** and **z** represent each independently a percentage of **m,** ranging from 0% to 100% of m, wherein x+y+z is equal to 100% of m;
**X** represents -COOH, alkyl, aryl;**Y** represents -(C=O)-O-L¹-R⁸, -(C=O)-S-L¹-R⁸, -(C=O)-NH(-L¹-R⁸) or -(C=S)-NH(-L¹-R⁸) wherein
   **L¹** represents a spacer selected from alkyl, alkene, aryl, arylalkyl, poly(ethylene glycol) or poly(propylene glycol) linking groups having 1 to 150 chain atoms, wherein the linking group can be optionally interrupted or terminated by one or more -O-, -S-, -NR⁹-, -CO-, - NHCO-, -CONH- or a combination thereof, wherein R⁹ is H or alkyl; **R⁸** represents a reactive group selected from N₃, amino, alkylamino, COOH, amide, maleimide, alkyne, SH, OH, ester, activated ester, activated carboxylic acid, halo, nitro, nitrile, isonitriles, acrylamide, aldehyde, ketone, acetals, ketals, anhydride, glutaric anhydride, succinic anhydride, maleic anhydride, thiocyanate, isothiocyanate, isocyanate, hydrazide, hydrazines, hydrazones, ethers, oxides, cyanates, diazo, diazonium, sulfides, disulfides, sulfoxides, sulfones, sulfonic acids, sulfinic acids, sulfates, sulfenic acids, amidines, imides, imidates, nitrones, hydroxylamines, oximes, hydroxamic acids, thiohydroxamic acids, allenes, ortho esters, sulfites, enamines, ynamines, ureas, pseudoureas, semicarbazides, carbodiimides, carbamates, imines, acetylene, olefins, polyenes, alkylacrylates, oxetane, ammoniums, oxoniums, phosphoniums, sulfoniums, positively charged metal complexes;
**Z** represents -(C=O)-O-L²-R¹⁰, -(C=O)-S-L²-R¹⁰, -(C=O)-NH(-L²-R¹⁰) or - (C=S)-NH(-L²-R¹⁰) wherein
   **L²** represents a single bond or a spacer selected from alkyl, alkene, aryl, arylalkyl, poly(ethylene glycol) or poly(propylene glycol) linking groups having 1 to 150 chain atoms, wherein the linking group can be optionally interrupted or terminated by one or more -O-, -S-, -NR⁹-, - CO-, -NHCO-, -CONH- or a combination thereof, wherein R⁹ is H or alkyl; optionally additionally comprising a residue of a reactive group through which L² is bonded to R¹⁰;
   **R¹⁰** represents a bioactive group selected from amino acid, peptide, protein, antibody, enzyme, polysaccharide, dextran, benzylguanine, lipid, lipid assembly, fatty acid, nucleoside, nucleotide, oligonucleotide, hapten, aptamer, ligand, substrate, biotin, avidin, streptavidin, synthetic polymer, poly(ethylene glycol), poly(propylene glycol), polymeric microparticle, nanoparticle, fluorophore, chromophore, radioisotope, macrocyclic complexes of radioisotope, and combinations thereof.

x+y+z is equal to 100% of m, at least one of x, y or z may be equal to 0% of m.According to one embodiment, the polymer of formula II is of formula II": 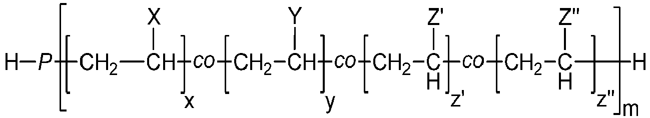 wherein,
**m, X** and **Y are** as defined in formula II;
**x, y, z'** and **z"** represent each independently a percentage of **m,** ranging from 0% to 100% of m, wherein x+y+z'+z" is equal to 100% of m;
**Z'** and **Z"** represent each independently -(C=O)-O-L²-R¹⁰, -(C=O)-S-L²-R¹⁰, -(C=O)-NH(-L²-R¹⁰) or -(C=S)-NH(-L²-R¹⁰) wherein
   **L²** represents a single bond or a spacer selected from alkyl, alkene, aryl, arylalkyl, poly(ethylene glycol) or poly(propylene glycol) linking groups having 1 to 150 chain atoms, wherein the linking group can be optionally interrupted or terminated by one or more -O-, -S-, -NR⁹-, - CO-, -NHCO-, -CONH- or a combination thereof, wherein R⁹ is H or alkyl; optionally additionally comprising a residue of a reactive group through which **L²** is bonded to R¹⁰;
   **R¹⁰** represents a bioactive group selected from amino acid, peptide, protein, antibody, affinity protein, enzyme, polysaccharide, dextran, benzylguanine, lipid, lipid assembly, fatty acid, nucleoside, nucleotide, oligonucleotide, hapten, aptamer, ligand, antibiotic, substrate, biotin, avidin, streptavidin, synthetic polymer, poly(ethylene glycol), poly(propylene glycol), polymeric microparticle, nanoparticle, fluorophore, chromophore, radioisotope, macrocyclic complexes of radioisotope, and combinations thereof.

According to one embodiment, one of Z' and Z" represents -(C=O)-O-L²-R¹⁰, -(C=O)-S-L²-R¹⁰, -(C=O)-NH(-L²-R¹⁰) or -(C=S)-NH(-L²-R¹⁰), wherein L² is a single bond and R¹⁰ is a poly(ethylene glycol) chain.

According to one embodiment, one of Z' and Z" represents -(C=O)-O-L²-R¹⁰, -(C=O)-S-L²-R¹⁰, -(C=O)-NH(-L²-R¹⁰) or -(C=S)-NH(-L²-R¹⁰), wherein L² is a poly(ethylene glycol) chain and R¹⁰ is a bioactive group different from a poly(ethylene glycol) chain.

According to one embodiment, Z' represents -(C=O)-O-L²-R¹⁰, -(C=O)-S-L²-R¹⁰, - (C=O)-NH(-L²-R¹⁰) or -(C=S)-NH(-L²-R¹⁰), wherein L² is a single bond and R¹⁰ is a poly(ethylene glycol) group; and Z" represents -(C=O)-O-L²-R¹⁰, -(C=O)-S-L²-R¹⁰,-(C=O)-NH(-L²-R¹⁰) or -(C=S)-NH(-L²-R¹⁰), wherein L² is a poly(ethylene glycol) chain and R¹⁰ is a bioactive group different from a poly(ethylene glycol) chain.

According to an embodiment, the polymer is random, alternate or block copolymer. In another embodiment, the ligand is a statistic copolymer. In another embodiment, said ligand is a random or block copolymer.

### B. Process for manufacturing the nanoassemblies of the invention

According to one embodiment, the manufacturing of the nanoassemblies of the present disclosure and of the invention is conducted using classical macroscopic means. According to an alternative embodiment, the manufacturing of the nanoassemblies of the present disclosure and of the invention is conducted using microfluidic means.

### B.1. Manufacturing of fluo@mag nanoassemblies (not according to the invention)

The present disclosure relates to the process for manufacturing the *fluo@mag* nanoassemblies of the disclosure.

According to one embodiment, the process of the disclosure comprises injecting a solution of fluorescent organic molecules into a dispersion of magnetic nanoparticles under stirring.

According to one embodiment, the solution of fluorescent organic molecules comprises an amount of fluorescent organic molecules ranging from 0.005 to 1% in weight relative to the total weight of the solution, preferably from 0.01 to 1% w/w, more preferably from 0.05 to 1% w/w.

According to one embodiment, the solvent of the solution of fluorescent organic molecules is an organic solvent, preferably an organic solvent miscible with water, preferably THF.

According to one embodiment, the dispersion of magnetic nanoparticles comprises an amount of magnetic nanoparticles ranging from 0.001 to 0.1% in weight relative to the total weight of the solution, preferably from 0.002 to 0.05% w/w, more preferably from 0.004 to 0.05%, more preferably from 0.004 to 0.01%.

According to one embodiment, the solvent of the dispersion of magnetic nanoparticles is an aqueous solvent, preferably water.

Preferably, the stirring during the manufacturing process is performed by means of a vortex.

### B.2. Manufacturing of fluo@mag@polymer nanoassemblies (according to the invention)

The present invention further relates to a process for the manufacturing of the *fluo@mag@polymer* nanoassemblies of the invention.

According to one embodiment, the process of manufacturing of the *fluo@mag@polymer* nanoassemblies comprises the manufacturing process of *fluo@mag* nanoassemblies as described above and a subsequent step of addition of a solution of polymer.

According to one embodiment, the process of manufacturing of the *fluo@mag@polymer* nanoassemblies comprises:
- injecting a solution of fluorescent organic molecules into a dispersion of magnetic nanoparticles under stirring, to form *fluo@mag* nanoassemblies; and
- adding the polymer.

According to one embodiment, the polymer is added under the form of a solid, preferably under the form of a powder. In an alternative embodiment, the polymer is added in solution, preferably an aqueous solution.

According to a preferred embodiment, the process further comprises a subsequent step of pH adjustment, preferably to a pH ranging from 6 to 12, preferably about pH 8. Preferably, pH adjustment is performed by the dropwise addition of a base, said base being preferably ammonium hydroxide, preferably the base is ammonium hydroxide at1 mol.L⁻¹.

According to a preferred embodiment, the process further comprises a subsequent step of dialysis, preferably a subsequent step of dialysis against Millipore water. Such a dialysis may enable to reach a pH of about 7.

The process may further comprise a step of lyophilization. Lyophilized nanoassemblies are easily stored for a prolonged period of time.

### B.3. Functionalization of fluo@mag@polymer nanoassemblies

According to one embodiment, the polymer used to form the *fluo@mag@polymer* nanoassemblies of the invention is a functionalized polymer. In this case, the formation of the *fluo@mag@polymer* nanoassemblies leads to functionalized *fluo@mag@polymer* nanoassemblies.

According to another embodiment, the polymer used to form the *fluo@mag@polymer* nanoassemblies of the invention is a functionalizable polymer. In this case, functionalization of the *fluo@mag@polymer* nanoassemblies may be performed after formation of the *fluo@mag@polymer* nanoassemblies by reacting the reacting groups of the polymer with a bioactive agent comprising a suitable reactive group. For example, when the polymer comprises carboxylic functions, the nanoassemblies may be functionalized after formation by reaction with a bioactive agent comprising an amine function through amide bond formation.

### C. Use of the nanoassemblies of the invention

The present invention further relates to the use of the nanoassemblies of the invention.

### Imaging

According to one embodiment, the nanoassemblies of the present disclosure and of the invention may be used for imaging, preferably medical imaging. Preferably, the nanoassemblies of the present disclosure and of the invention are used for bimodal imaging, namely for fluorescence imaging and magnetic resonance imaging.

In one embodiment, imaging using the nanoassemblies of the present disclosure and of the invention may be performed *in vivo.* In another embodiment, imaging using the nanoassemblies of the present disclosure and of the invention may be performed *in vitro.* In another embodiment, imaging using the nanoassemblies of the present disclosure and of the invention may be performed *ex vivo.*

The present disclosure further relates to a method of medical imaging which comprises administering to a patient in need thereof the nanoassemblies according to the present disclosure and recording fluorescence and magnetic signals.

The invention further relates to a method of medical imaging which comprises administering to a patient in need thereof the nanoassemblies according to the invention and recording fluorescence and magnetic signals.

According to one embodiment, the nanoassemblies of the present disclosure and of the invention may be used for diagnosis purposes.

According to one embodiment, the nanoassemblies of the present disclosure and of the invention may be used for assessing, *in vitro* or *in vivo,* the biodistribution of substances of interest.

The present disclosure also relates to the use of the nanoassemblies of the present disclosure in correlative microscopy and/or in electronic microscopy.

The invention also relates to the use of the nanoassemblies of the invention in correlative microscopy and/or in electronic microscopy.

### Therapy

According to one embodiment, the nanoassemblies of the present disclosure and of the invention may be used for therapy.

According to one embodiment, the nanoassemblies of the present disclosure and of the invention may comprise a therapeutically active agent. In this case, the nanoassemblies may act as a delivery means of said active agent.

Magnetic properties of the nanoassemblies of the present disclosure and of the invention may be used to generate hyperthermia. Therefore, the nanoassemblies of the present disclosure and of the invention may be used to treat diseases by hyperthermia. Especially, hyperthermia may be used to destroy tumors. The nanoassemblies of the present disclosure and of the invention were tested for their ability to induce hyperthermia under external alternating magnetic field. A significant temperature increase was obtained with a solution of *fluo@mag@PAA* nanoassemblies in water upon applying an alternating magnetic field. A significant temperature increase was also observed in presence of mesothelium cells.

In the case of nanoassemblies bearing a polymer protective shell, the polymer may be functionalized by targeting agents and/or pharmaceutically active agents.

The present disclosure relates to a pharmaceutical composition comprising a nanoassembly according to the present disclosure, optionally functionalized with a pharmaceutically active agent, in combination with at least one pharmaceutically acceptable vehicle.

The present invention relates to a pharmaceutical composition comprising a nanoassembly according to the invention, optionally functionalized with a pharmaceutically active agent, in combination with at least one pharmaceutically acceptable vehicle.

The present disclosure also relates to a medicament comprising a nanoassembly according the present disclosure, said nanoassembly being optionally functionalized with a pharmaceutically active agent.

The invention also relates to a medicament comprising a nanoassembly according to the invention, said nanoassembly being optionally functionalized with a pharmaceutically active agent.

The present disclosure also relates to a nanoassembly according to the present disclosure for use as a medicament, preferably for use in the treatment of and/or prevention of cancer or infections by pathogens.

The invention also relates to a nanoassembly according to the invention for use as a medicament, preferably for use in the treatment of and/or prevention of cancer or infections by pathogens.

According to one embodiment, "infections by pathogens" refers to infections caused by *Staphylococcus aureus, Pseudomonas aeruginosa, Plasmodium falciparum* or *Streptococcus pneumonia.*

According to one embodiment, the nanoassembly of the present disclosure and of the invention is for use in the treatment and/or prevention of cancer or of infections by pathogens. According to one embodiment, the nanoassembly of the present disclosure and of the invention is for use in the treatment and/or prevention of cancer.

According to one embodiment, the nanoassembly of the present disclosure and of the invention, preferably the functionalized nanoassembly of the present disclosure and of the invention, is for use as an active therapeutic substance.

### Cell sorting

The present disclosure further relates to the use of the nanoassembly of the present disclosure for cell sorting, involving magnetic and/or fluorescent properties of the nanoassemblies of the invention.

The present invention further relates to the use of the nanoassembly of the invention for cell sorting, involving magnetic and/or fluorescent properties of the nanoassemblies of the invention.

Especially, fluorescent properties may be used to sort cells by flow cytometry. Flow cytometry methods are known by those skilled in the art. For example a flow cytometry method may be FACS (fluorescence-activated cell sorting).

Magnetic properties of the nanoassemblies of the present disclosure and of the invention may also be used for magnetic cell sorting.

According to one embodiment, the nanoassemblies of the present disclosure and of the invention are used for fluorescent cell sorting. According to another embodiment, the nanoassemblies of the present disclosure and of the invention are used for magnetic cell sorting. According to one embodiment, the nanoassemblies of the present disclosure and of the invention are used for fluorescent and magnetic cell sorting.

### Ex vivo sensing and/or extraction

The present disclosure further relates to the use of the nanoassemblies of the present disclosure for *ex vivo* sensing.

The present invention further relates to the use of the nanoassemblies of the invention for *ex vivo* sensing.

Especially, the nanoassemblies of the present disclosure and of the invention may be used to detect compounds and/or biological targets in physiological medium. The nanoassemblies of the present disclosure and of the invention may also be used to extract compounds and/or biological targets from physiological medium. According to one embodiment, the nanoassemblies of the present disclosure and of the invention are used to extract and detect molecules and/or biological targets from physiological medium.

According to one embodiment, the biological target is selected from pathogens, proteins, antibodies, virus and xenobiotics, preferably pathogens. By "pathogen" it is herein referred to a microorganism, such as for example virus, bacterium, prion, fungus or protozoan, that causes disease in its host.

According to one embodiment, the physiological medium is selected from plasma, serum, blood, urine and lymph, preferably blood.

For *ex vivo* sensing, the polymer of the nanoassembly is preferably functionalized with a bioactive group enabling an interaction with the compounds and/or biological targets to be detected.

*Ex vivo* sensing may be performed by contacting the nanoassemblies of the present disclosure and of the invention with the physiological medium to be analyzed. The bioactive group functionalizing the nanoassemblies then interacts with the compounds and/or biological targets to be detected. Such interaction may lead to formation of aggregates of nanoassemblies and targets. Trapping may then be conducted by applying a magnetic field, taking advantage of the magnetic properties of the nanoassemblies of the present disclosure and of the invention. The remaining medium may then be removed by washing. After magnetic release, detection may be performed by fluorescence.

### Kit

The present disclosure further relates to a kit comprising nanoassemblies according to the present disclosure. The present invention further relates to a kit comprising nanoassemblies according to the invention. The kit may further comprise one or more additional components, such as for example a redispersion solution (for example: buffer, serum, culture cell medium). According to an embodiment, the kit further comprises calibration standards.

According to one embodiment, the nanoassemblies of the present disclosure and of the invention may be present in the kit associated with a surface, such as for example a chip, microplate well, or other solid or semi-solid matrix.

### BRIEF DESCRIPTION OF THE DRAWINGS

**Figure 1** is a schematic representation of the *fluo@mag* nanoassemblies which is not part of the invention and of their process of manufacturing.
**Figure 2** is a schematic representation of the *fluo@mag@polymer* nanoassemblies of the invention and of their process of manufacturing.
**Figure 3** is a TEM image of *fluo@mag@PAA* nanoassemblies after synthesis. Inlet: zoom-in with a 20 nm scale bar.
**Figure 4** is the evolution of the transverse relaxivities R2 of *fluo@mag@PAA* nanoassemblies (D_{H} = 150 nm; black dot labels) and dispersed iron oxide nanoparticles coated with PAA (mag@PAA: D_{H} = 7 nm; white square labels) as a function of frequency. Vertical bars represent measure errors.
**Figure 5** is the evolution of longitudinal relaxivities R¹ of *fluo@mag@PAA* nanoassemblies (D_{H} = 150 nm; black dot labels) and dispersed γ-Fe₂O₃ nanoparticles coated with PAA (mag@PAA: D_{H} = 7 nm; white square labels) as a function of frequency. Vertical bars represent measure errors.
**Figure 6** UV-vis absorption spectra in water of the fluorescent organic nanoparticles (fluo absorption) and γ-Fe₂O₃ nanoparticles (γ-Fe₂O3 absorption) and emission spectra (λ_{exc} = 450 nm) of the fluorescent organic nanoparticles (fluo emission) of *fluo@mag@PAA* nanoassemblies (fluo@mag@PAA emission).
**Figure 7** is fluorescence confocal laser scanning microscopy images of HEK cells incubated with *fluo@mag@PAA nanoassemblies* at: a) a 3 µM concentration of fluorescent molecules with no staining agent *λ*_{exc} = 488 nm, *λ*ₑₘ > 510 nm), b) a 1 µM concentration of fluorescent molecules and post-treated with a nucleus staining agent (Hoechst 33342) (λ_{exc} = 405 nm, λₑₘ > 510 nm) (objective ×63, NA 1.4).
**Figure 8** is TEM images of internalized nanoparticles in HEK cells (70 nm-thin section). a) General view. b) Zoom-in view. c) and d) Endosomes trapping the *fluo@mag@PAA* nanoassemblies.
**Figure 9** is T2*-weighted MR images of mouse's liver. a) before the injection, b) 25 min after the injection, c) 230 min. after the injection. The white circle corresponds to a water magnetic resonance phantom.
**Figure 10** is T2*-weighted MR images of mouse's spleen. a) before the injection, b) 25 min after the injection. The white circle corresponds to a water magnetic resonance phantom.
**Figure 11** is TEM images of mouse's liver tissue after intravenous injections of the *fluo@mag@PAA nanoassemblies* of the invention.
**Figure 12** Comparative emission spectra (*λ*_{exc} = 350 nm) and fluorescence decays (*λ*ₑₘ = 340 nm, *λ*ₑₘ = 440 nm) in water of 7-amino-4-methylcoumarine (AC)*, fluo@mag@PAA-AC* nanoassemblies issued from the surface functionalization of *fluo@mag@PAA* with AC, and mag@PAA-AC issued from the surface functionalization of *mag@PAA* with AC.

### EXAMPLES

The present invention is further illustrated by the following examples.

### MATERIAL

All chemical reagents and solvents were purchased from commercial sources (Aldrich, Acros, SDS) and used as received. Spectroscopic grade solvents purchased from Aldrich were used for spectroscopic measurements.

### ABBREVIATIONS

- **°C**: Celsius degree
- **cm**: centimeter
- **DAPI**: 4', 6'-diamidino-2-phenylindole
- **D_{H}**: Hydrodynamic diameter
- **DLS**: Dynamic Light Scattering
- **DMEM**: Dulbecco/Vogt modified Eagle's minimal essential medium
- **DMSO**: Dimethylsulfoxide
- **emu**: 1 emu = 10⁻³ A m⁻² = 10⁻³ J T⁻¹ features the unit of the magnetic moment
- **ENH**: Contrast magnetic resonance enhancement
- **G**: Gauss
- **h**: hour
- **HBSS**: Hank's Balanced Salt Solution
- **HEK**: Human Embryonic Kidney cell
- **K**: Kelvin
- **Kg**: kilogram
- **λ**: Wavelength
- **LSM**: Laser Scanning Microscope
- **M**: mole per liter
- **MDa**: Mega Dalton
- **MDA**: Cell derived from metastatic site in human mammary gland/breast
- **MHz**: Mega Hertz
- **min**: minute
- **mL**: milliliter
- **MRI**: Magnetic Resonance Imaging
- **Ms**: saturation magnetization
- **MTT**: 3-(4, 5-dimethylthiazol-2-yl)-2, 5-diphenyltetrazolium bromide
- **ms**: millisecond
- **nm**: nanometer
- **NMR**: Nuclear Magnetic Resonance
- **NPs**: Nanoparticles
- **OCT**: Embedding matrix for cryotomy
- **PAA**: Poly(acrylic acid)
- **PBS**: phosphate buffered saline
- **R1**: longitudinal relaxivity
- **R2**: transverse relaxivity
- **RPMI**: Roswell Park Memorial Institute medium
- **s**: second
- **T**: Tesla
- **T1**: Longitudinal relaxation time
- **T2**: Transverse relaxation time
- **TE**: echo time
- **TEM**: Transmission Electron Microscopy
- **THF**: Tetrahydrofuran
- **TR**: repetition time
- **µM**: micromole per liter
- **UV**: ultraviolet
- **v**: frequency

### PART A (reference example) - MAGNETIC-FLUORESCENT NANOASSEMBLIES

### A.1. SYNTHETIC PROCEDURES

### A.1.1 Fluorescent organic molecules

Fluorescent molecules (Ia) and (Ib) were obtained as described in Faucon et al., J. Mater. Chem. C., 2013, 1, 3879-3886.

Stock solutions of the fluorescent molecules (Ia) and (Ib) in spectrophotometric grade THF were prepared at concentrations ranging from 0.05 to 1 wt%.

### A.1.2. Magnetic nanoparticles

***Nitrate-stabilized magnetic nanoparticles.*** Maghemite γ-Fe₃O₃ nanoparticles were prepared according to a procedure described by Massart et al (IEEE Trans. Magn., 1981, 17, 1247-1248). Briefly, Fe(II) and Fe(III) salts were mixed in dilute hydrochloric acid. Quick alkalinization of the medium by adding concentrated ammonia enables the coprecipitation of magnetite Fe₃O₄ nanoparticles which were separated. The acidification of γ-Fe₃O₄ with nitric acid, followed by chemical oxidation with ferric nitrate at 80 °C, yielded γ-Fe₃O₃ nanoparticles. After magnetic decantation, the red precipitate was dispersed in nitric acid (pH = 1.2) since nitrate ions act as stabilizing counter-ions of the positively charged surface of the colloidal dispersion (ζ = +25 mV).

***Magnetite nanoparticles.*** The synthesis was performed according by adapting Santoyo et al. (Chem. Mater. 2011, 23, 1379-1386). Two solutions of iron salts were prepared: FeCl₃, 6H₂O, 1 mol.L⁻¹ in HCl 2 mol.L⁻¹ (10 mL) and FeCl₂, 4H₂O, 2 mol.L⁻¹ in HCl 2 mol.L⁻¹ (2.5 mL). After thorough deoxygenation with nitrogen for 2 h, both solutions were mixed and heated up to 70 °C under argon atmosphere under vigorous stirring. A solution of tetrapropylammonium hydroxide (1 mol.L⁻¹) was then injected at a 0.7 mL.min⁻¹ rate, and vigorous stirring was continued for an extra 20 min. The resulting nanoparticles were washed twice by magnetic decantation and dialyzed against Millipore water until neutral pH was obtained. In order to obtain a stable dispersion of magnetite in acidic media, the nanoparticle were sonicated in 2 M HNO₃ for 15 min and then washed one time by magnetic decantation with 0.1 M of HNO₃. A colloidal dispersion is obtained after stirring the nanoparticles for few days which was eventually filtered through 0.2 µm to remove larger nano-objects. After the previous dialysis step, one drop of amine was added to provide a stable dispersion of magnetite nanoparticles Fe₃O₄ in neutral water, which was eventually filtered through 0.2 µm to remove larger nano-objects.

### A.1.3. Fluo@mag nanoassemblies formation

A 0.1 wt% stock solution of fluorescent molecules in THF (50 µL) was injected into a 0.006 wt% solution of maghemite nanoparticles (2.5 mL) stirred by means of a vortex. When a nitric acid solution of magnetic nanoparticles was used, formation of hybrid nanoparticles, dubbed *fluo@mag* and comprising fluorescent molecules and iron oxide nanoparticles, is instantaneous.

### A.2. PHYSICO-CHEMICAL CHARACTERIZATIONS

### A.2.1. Nanoassembly characterizations

***Methods.*** The hydrodynamic diameter and size distribution of the nanoassemblies were determined by dynamic light scattering (DLS) by means of a nanoparticle size analyzer Zetasizer Nano ZS ZEN 3600 (Malvern Instruments) equipped with a 4 mW He-Ne laser, operating at 633 nm, and a photomultiplier detector collecting back-scattered light at an angle of 175°. Measurements were carried out at 20 °C on aqueous solutions of nanoassemblies. For each sample, intensity measurements were carried out in a multi-acquisition mode implying automatically adjusted correlograms, and averaged measurements on 3 acquisitions. Nanoparticle mean sizes and distribution widths were obtained by fitting each correlogram with a Cumulants algorithm.

Measurements of surface potential ζ were carried out by means of a Zetasizer Nano ZS ZEN 3600 (Malvern). The samples were placed in plastic cells. Several measurements were realized for each sample according to a predefined operating procedure.

The nanoassembly morphology was investigated by transmission electron microscopy (TEM, Hitachi HF2000-FEG). Solutions of nanoassemblies were deposited onto holey carbon coated copper grids (300 mesh) for all compounds except for the *fluo@mag* nanoparticles prepared from naked γ-Fe₂O₃ nanoparticles dispersed in dilute nitric acid, which were deposited onto holey carbon-coated gold grids (300 mesh).

***Results.*** DLS and TEM measurements of *fluo@mag* nanoassemblies invariably yielded a mean diameter of 90-100 nm with a quite narrow size distribution. The colloidal suspension of nanoassemblies was stable in dilute nitric acid over months due to the negatively charged surface (ζ = -33 mV) as measured by zetametry. TEM imaging revealed a raspberry-like assembly where γ-Fe₂O₃ nanoparticles contacted an organic nanosphere resulting from the self-aggregation of fluorescent molecules upon phase separation in aqueous solution.

### 2.2. Photophysical measurements

***Methods.*** UV-visible absorption spectra were recorded using a Varian Model Cary 5E spectrophotometer, using an integrating sphere DRA 2500. Corrected emission spectra were obtained using Jobin-Yvon. Inc spectrofluorimeter (Fluorolog 2). Fluorescence quantum yields *Φ*_{f} in solution were determined from Coumarine 540 A in EtOH (*Φ*_{f} = 0.38). Fluorescence intensity decays were measured at 580 nm using a monochromator (Hamamatsu MCP R3809U photomultiplier) by the time-correlated single-photon counting method (TCSPC) with a femtosecond laser excitation at 450 nm provided by a Spectra-Physics setup (Titanium-Sapphire Tsunami laser pumped by a doubled YAG laser (Millennia), pumped itself by a two-laser diode array, and doubling LBO crystals). Light pulses at 900 nm were selected by optoacoustic crystals at a repetition rate of 4 MHz, and then doubled at 450 nm.

**Table 1. Photophysical characteristics of fluo@mag nanoassemblies dispersed in water.**

| λₘₐₓ(abs) [nm] | λₘₐₓ(em) [nm] | *τ*(*f*_{¡})¹ [ns] | <*τ*_{S}>² [ns] |
|---|---|---|---|
| 443 | 604 | 0.77 (34%), 0.24 (48%), 0.08 (14%), 0.04 (4 %) | 0.39 |

| | | | |
|---|---|---|---|
| *¹After laser excitation at 450 nm and detection at 580 nm.* *²The intensity averaged time constant <τ_{S}> and normalized time fractional amplitude fᵢ are calculated from global analysis using a multiexponentialfit I(t)* = *∑ᵢ aᵢexp*(- *tlτᵢ) with aᵢ pre-exponential factor. The intensity-averaged time constant <τₛ> is defined as 〈τ_{S}〉* = ∑*ᵢfᵢτᵢ with fᵢ* = *aᵢτᵢ*/∑*ⱼaⱼτⱼ.* | | | |

***Results.*** The steady-state emission spectrum of the resulting *fluo@mag* nanoassemblies appeared very similar to that of fluorescent nanospheres in terms of energy (5 nm hypsochromic shift) and intensity (*Φ*_{f} = 0.01). Such a strong similarity stems from the main contribution of the fluorescent molecules comprised in the core, isolated from the interface. Partial emission quenching of the fluorescent nanospheres occurred via vibrational coupling between water and fluorescent molecules at the interface. For *fluo@mag* nanoassemblies, such quenching operates from electron transfer from the peripheral fluorescent molecules to the contacting iron oxide nanoparticles, which extends only to a few nanometers, with no effect on the fluorescent molecules inside the core. Shortening of the fluorescence decay was indeed observed by time-resolved fluorescence measurements. The longer lifetime constant, assessed at 1.49 ns for the fluorescent nanospheres, decreased to 0.08 ns for the *fluo@mag* nanoassemblies. These results emphasize the adopted approach of reverse architectures, insulating fluorescent molecules from iron oxide nanoparticles, known as strong emission quenchers.

### 2.3. Magnetic measurements

***Methods.*** Temperature dependent magnetization experiments on the colloidal suspension of fluo@mag nanoparticles ([Fe] ∼ 6.5×10⁻⁵ mol.L⁻¹) were collected with a Quantum Design MPMS-5 S SQUID magnetometer working at the temperature of 298 K and in the magnetic field range 0-2 T. The samples were diluted enough to avoid magnetic dipole-dipole interactions. The hysteresis loops were performed at 298 K in ZFC samples.

***Results.*** The magnetization curve as a function of the applied magnetic field at 300 K shows very small coercivity, typical of superparamagnetic nanoparticles. The saturation magnetization values Ms were found to be 22 emu.g⁻¹ for *fluo@mag* nanoassemblies. The reduced Ms values relative to bulk maghemite (75-80 emu.g⁻¹) could be due to an increase in the surface anisotropy induced by chemisorption of the fluorescent molecules at the surface. Taking into account the iron mass determined from elemental analyses, fit of the magnetization curve provides a size distribution peaking at 7.29 nm (width σ = 0.25) in fair agreement with the mean diameter of the initial nanoparticles (*D*_{H} = 8.1 nm). Such values tend to indicate that γ-Fe₂O₃ nanoparticles still keep their superparamagnetic behaviour when adsorbed.

### PART B - POLYMER NANOASSEMBLY SYSTEMS

Coating the *fluo@mag* nanoassemblies with an appropriate polyelectrolyte (i.e. polymer) leads to stable colloidal solutions of nanoassemblies undergoing neither aggregation nor dissociation in aqueous media, which is suitable for *in vitro* and *in vivo* applications. Their subsequent lyophilization yields fine powders for a long-term storage which can be redispersed in media of various chemical natures and ionic strengths with no loss of the structural and functional properties.

### B.1. SYNTHETIC PROCEDURES

The protocols below describe the stabilization process of the fluorescent and magnetic *fluo@mag* nanoassemblies by using the PAA polyelectrolyte (M_{w} = 1.8-2.2 kDa) as a polymer. Lyophilization of the stabilized *fluo@mag@PAA* nanoassemblies for biological uses in various media of interest is described.

### B.1.1. Synthesis of fluo@mag@PAA nanoassemblies

***With maghemite nanoparticles.*** A solution of fluorescent compound *(Ib)* dissolved in THF (50 µL, 0.1 wt. %) was added under vigorous stirring to a solution of maghemite nanoparticles in nitric acid (2.5 mL, 0.006 wt. %, pH = 1.2). After a few seconds, the magnetofluorescent nanoassemblies *(Ib)-fluo@maghemite* were formed. Poly(acrylic acid) (2.1 kDa, 5 mg) was added as powder; ammonium hydroxide (1 mol.L⁻¹) was added dropwise under stirring until pH = 9 was reached. The resulting translucent solution was allowed to stir for a further 30 min and dialyzed using a Spectra Por membrane (Standard Grade Regenerated Cellulose; cut-off: 8-10 kDa) against Millipore water (600 mL) over 24 h until the final pH solution of the *fluo@mag@PAA* nanoassemblies **(1)** reached a value of 7.

***With magnetite nanoparticles.*** A solution of compound *(Ib)* dissolved in THF (50 µL, 0.1 wt. %) was added under vigorous stirring to a solution of magnetite nanoparticles in water (2.5 mL, 0.006 wt. %). After a few hours (usually two hours), the magnetofluorescent nanoassemblies *(Ib)-fluo@magnetite* formed. Polyacrylic acid (2.1 kDa, 5 mg) was added as a powder and then, ammonium hydroxide (1 mol.L⁻¹) was added dropwise under stirring until pH = 9 was reached. The resulting translucent solution was allowed to stir for a further 30 min and dialyzed using a SpectraPor membrane (Standard Grade Regenerated Cellulose; cut-off: 8-10 kDa) against Millipore water (600 mL) over 24 h until the final pH solution of the *fluo@mag@PAA* nanoassemblies **(2)** reaches a value of 7. The suspension of nanoassemblies was finally stored at 4°C.

### B.1.2. Lyophilization

In order to store the resulting *fluo@mag@PAA* nanoassemblies over a long period of time in the solid state, volumes (1 mL to 3 mL) of nanoparticle solutions were placed in glass vials such that the height of the liquid was not higher than 1 cm. The solution was allowed to freeze using liquid nitrogen. Lyophilization was performed over 9 h to 12 h to yield a dark red power that was stored at -18 °C under nitrogen atmosphere.

### B.1.3. Re-dispersion procedures

Nanoassembly redispersion was easily performed by adding the adequate solvent (water, physiological media, alcoholic solvents such as ethanol) to the lyophilized sample. No ultrasound treatment was required since the nanoassemblies undergo no aggregation upon redispersion. Iron concentration in the range of 0.6-3×10⁻³ mol.L⁻¹, even in the range of 0.6-7×10⁻³ mol.L⁻¹, could be obtained depending of the added amount of re-dispersing solvent.

### B.2. PHYSICO-CHEMICAL CHARACTERIZATION

The characterizations of *fluo@mag@PAA* nanoassemblies **(1)** in terms of size, surface potential, composition, photophysical and relaxivity properties are described. The stability of the colloidal suspensions as a function of time and the nature of solvent was checked by DLS, TEM and UV-vis absorption measurements (UV-visible absorption spectra were recorded using a Agilent Model Cary 5E spectrophotometer, equipped with an integrating sphere DRA 2500). The composition (number of fluorescent molecules in the core and magnetic nanoparticles of the shell) has been determined by both mass spectrometry and magnetic sedimentation.

DLS and TEM measurements of *fluo@mag@PAA* nanoassemblies **(1)** yield a mean diameter of 120-150 nm. DLS measurements, performed in water, generally display slightly larger diameters due to the first water solvation sphere. The nanoassemblies are stable in water over a period of 5 months at room temperature and over almost a year at 4 °C. The size distribution varies little after lyophilization and redispersion in various media (water, HBSS, PBS, ethanol) as demonstrated by DLS and UV-vis absorption measurements, in accord with parallel DLS and TEM analyses. The *fluo@mag@PAA* nanossembly **(1)** cohesion, ensured by the PAA coating, is also demonstrated by means of ultra-sounds which do not dissociate the nanoassemblies. Mass spectrometry analyses and magnetic sedimentation, enabling the composition determination, show convergent data: the core was made of ∼10⁵ organic molecules (in agreement with previous results involving single nanoparticle photoabsorption measurements), surrounded by a shell of ∼10⁴ maghemite nanoparticles. Relaxivity measurements demonstrate large cooperativity effects between the vicinal iron oxide nanoparticles assembled in the magnetic shell. The threefold increase in the r₂/r₁ ratio observed for the nanoassemblies allows for highly contrasted T2-weighted MRI. Finally, steady-state and time-resolved fluorescence measurements show that the fluorescence signal is not affected by the surrounding media as expected from the protecting role played by the maghemite nanoparticle shell.

### B.2.1. Size characterizations

Size characterizations (DLS and TEM measurements) of the *fluo@mag@PAA* nanoassemblies **(1)** were carried out after formation, after the lyophilization-redispersion process and after having been subjected to ultra-sounds. After redispersion, no agglomeration is observed by DLS even for solutions with concentrations higher than those of the initial solution before lyophilization. Moreover no nanoassembly dissociation is observed after ultra-sounds.

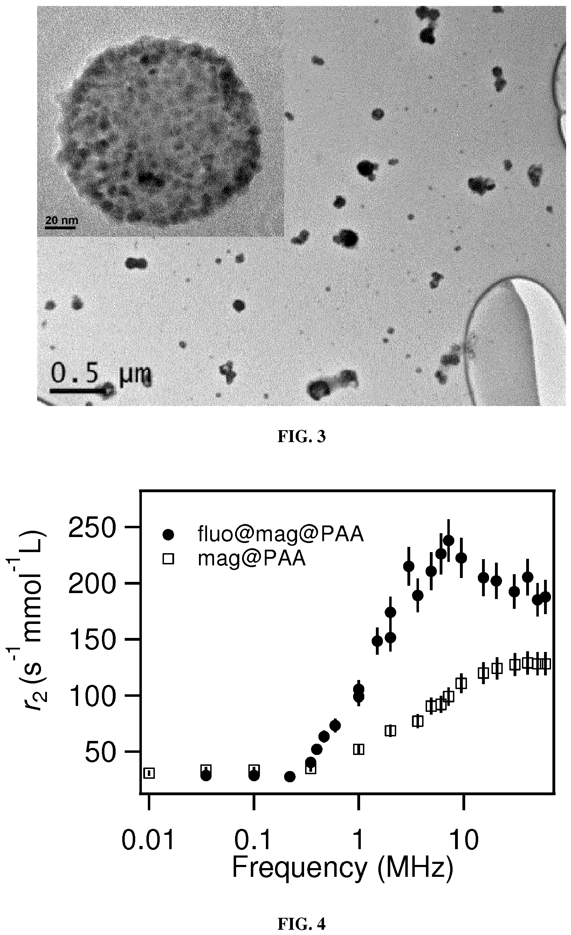

The assemblies characterized by TEM, were deposited on holey carbon grids and dried at room temperature. Observations **(****Figure 3****)** were performed with a MO-Jeol MET 1230, working at a 80 kV voltage.

### B.2.2. Colloidal stability

The stability of *fluo@mag@PAA* nanoassemblies **(1)** was studied as a function of time and in distinct solutions with various pHs and ionic strengths.

DLS and TEM measurements of nanoassemblies dispersed in Millipore water were conducted right after the synthesis and after a few months. The results feature the high stability of the nanoassembly structure (TEM - **Figure 3**).

UV-vis absorption analyses were also performed at 450 nm as a function of time to follow the colloidal stability. In the case of unstable dispersions of nanoparticles, sedimentation is usually observed with a loss of absorbance. Absorbance decreases by less than 2% after 11 days, proving the highly stable colloidal character of the nanoassemblies, be they placed in water or redispersed in HBSS or PBS buffer.

Measurements of the hydrodynamic diameter distribution of *fluo@mag@PAA* nanoassemblies were also performed in various media and for various durations of storage at room temperature to assess colloidal stability. The previously lyophilized nanoassemblies were redispersed in pure water or in a physiological medium composed of PBS and 10% of bovin serum, with no significant change in the distribution of hydrodynamic diameter. A slight increase in the mean diameter is observed when the nanoassemblies were dispersed in a medium composed of bovin serum only (100%), due to non-specific adsorption of proteins on the nanoassembly surface. After 24 h, neither decantation nor precipitation was further observed, despite the slight increase in the hydrodynamic diameter. Consequently, the nanoassemblies exhibit unique stability in highly concentrated serum (up to 100 %).

The nanoassemblies are stable over a large pH range (3-12) due to a significant surface potential ζ < -31 mV, causing efficient electrostatic repulsions between the nanoassemblies. As a consequence, no aggregation could also be observed in media of varying ionic strength up to 0.3 mol.L⁻¹, which is beneficial for biological applications dealing with media usually with high ionic strength.

### B.2.3. Composition determination

Studies were conducted to determine the amount of iron oxide NPs on the surface of the organic core by means of mass spectrometry and magnetic sedimentation respectively. Titrations of iron content of the nanoassemblies may also be performed by resorting to ICP-AES measurements (Inductive Coupled Plasma - Atomic Emission Spectrometry).

Mass spectrometry experiments were realized according to the method described in Doussineau et al., Rapi. Commun, Mass Spectrom, 2011, 25, 617-623*.* The molar mass distributions for the fluorescent nanospheres and the *fluo@mag@PAA* nanoassemblies **(1)** were recorded. The noticeable increase in weight (namely 86 MDa) when going from fluorescent organic nanospheres to *fluo@mag@PAA* nanoassemblies **(1)** can be mainly correlated to the amount of iron oxide nanoparticles, assessed around 2 × 10⁴ nanoparticles per nanoassembly. These measurements also confirm the amount of fluorescent molecules per organic core, valued to be around 10⁵ fluorescent molecules. Magnetic decantation experiments were carried out to analyze the time-evolution of the normalized absorbance at 552 nm and 446 nm. Magnetic sedimentation was performed with solutions of maghemite nanoparticles dispersed in dilute nitric acid (pH =1.2) and solutions of *fluo@mag@PAA* nanoassemblies **(1)** dispersed in water.

Comparative measurements of the magnetic sedimentation of single maghemite nanoparticle dispersions and *fluo@mag@PAA* nanoassemblies **(1)** dispersions show stronger sedimentation for the latter in accord to their higher magnetic content. Modeling the absorbance decay indicated that the magnetic shell of the nanoassembly comprised ∼10⁴ maghemite nanoparticles, in agreement with the data obtained by mass spectrometry.

### B.2.4. Relaxivity measurements

Relaxivity measurements (¹H NMR longitudinal T1, and transverse T2 relaxation times) were conducted for the *fluo@mag@PAA* nanoassemblies **(1)** in diluted solutions.

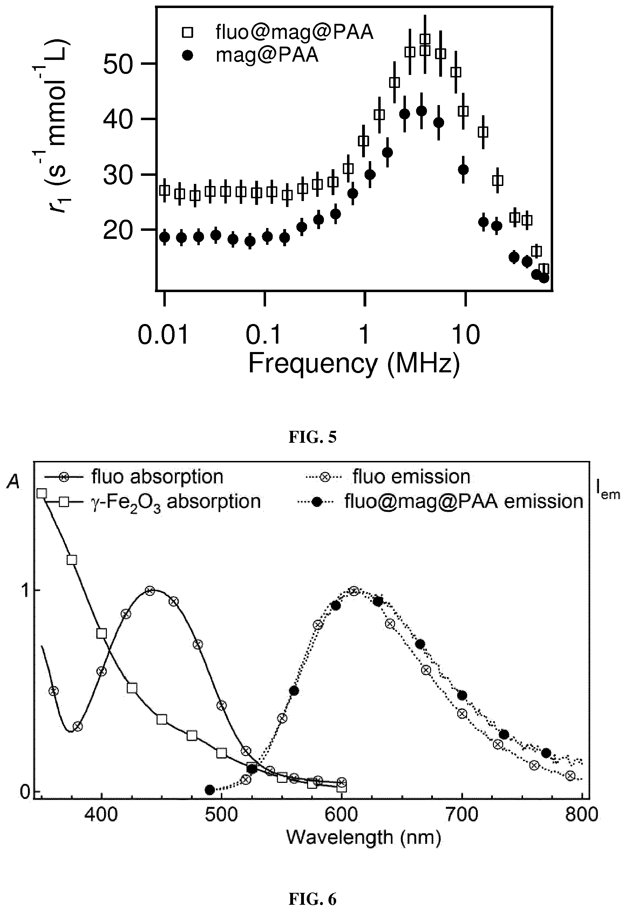

Measurements of ¹H longitudinal T1 and transverse T2 relaxation times at different frequencies v at room temperature were performed on diluted solutions of samples (at v = 200 and 300MHz), in the range of 10 kHz ≤ v ≤ 212 MHz for T1 and 15 MHz ≤ v ≤ 60 MHz for T2. Proton T1 and T2 relaxation time measurements were performed on aqueous solutions with different concentrations of nanoassemblies at room temperature (300 K) under an applied magnetic field of 4.7 T using a Tecmag Apollo spectrometer operating at 200 MHz. The rates observed for both longitudinal and transverse relaxations showed a linear dependence according to the nanoparticle concentration. Using the inversion-recovery and spin echo (Carr-Purcell Meiboom Gill) sequences to measure T1 and T2, the longitudinal r₁ and transverse r2 relaxivities were calculated from the slope of the plot of 1/T1 and 1/T2 curves vs nanoparticle concentration. **Figures 4** and **5** feature the transverse and longitudinal relaxivities respectively, as a function of frequency, of *fluo@mag@PAA* nanoassemblies **(1)** and dispersed iron oxide nanoparticles coated with PAA.

Higher values of transverse relaxivity r2 are obtained for *fluo@mag@PAA* nanoassemblies **(1)** compared to those of dispersed iron oxide nanoparticles. The longitudinal relaxivity r₁ varies in the opposite trend. The high values of T2 and the large increase in the r₂/r₁ ratio for the nanoassemblies, related to the T2 weighted MRI, demonstrate the very positive additive effects between the iron oxide nanoparticles at the surface of the nanoassemblies. Such additivity does not exist for isolated iron nanoparticles serving as a reference.

### B.2.5. Photophysical properties

Steady-state absorption and emission measurements **(****Figure 6****),** time-resolved fluorescence measurements, and wide-field fluorescence microscopy of *fluo@mag@PAA* nanoassemblies **(1)** dispersed in various media were realized to characterize their emission intensity and spectral range.

**Table 2. Photophysical characteristics of fluo@mag@PAA nanoassemblies (1) dispersed in water.**

| *λ*ₘₐₓ(abs) [nm] | *λ*ₘₐₓ(em) [nm] | *τ*(*f*ᵢ)¹ [ns] | <*τ*_{S}>² [ns] |
|---|---|---|---|
| 443 | 604 | 0.7 (29%), 0.2 (52%), 0.04 (19%) | 0.31 |

| | | | |
|---|---|---|---|
| *¹After laser excitation at 450 nm* and *detection at 580 nm.* *²The intensity averaged time constant <τ_{S}> and normalized time fractional amplitude fᵢ are calculated from global analysis using a multiexponentialfit I*(*t*) = ∑*ᵢ aᵢexp* (- *t*/*τᵢ*) *with aᵢ pre-exponential factor. The intensity-averaged time constant <τₛ> is defined as* 〈*τₛ〉* = ∑*ᵢfᵢτᵢ with fᵢ* = *aᵢτᵢ*/∑*ⱼ aⱼτⱼ.* | | | |

No significant effect of the presence of the PAA coating on the emission properties could be observed compared to the non-stabilized *fluo@mag* nanoparticles. The fluorescence signal is also insensitive to the nature of the surrounding solutions. This permits accurate detection of the nanoparticles in biological media, requesting no change in the fluorescence intensity or color unless specifically expected. Efficient fluorescence signal is detected upon exciting the *fluo@mag@PAA* in the absorption band of the fluorescent core. The red-shifted emission compared to the maghemite nanoparticle absorption band avoids deleterious emission reabsorption by the maghemite nanoparticles **(****Figure 6****),** although a small decrease in the emission intensity of the core compared to that of neat fluorescent nanospheres could be noticed. This effect is due to electron transfer from the core to iron (III) ions of the maghemite nanoparticles. Such an emission quenching is actually much stronger for the normal or doped architectures usually reported in literature, where each peripheral fluorescent unit is in direct contact with the iron oxide nanoparticles.

### B.3. IN VITRO EXPERIMENT

Two different lines of human cells (HEK 293 and MDA-MB-468) are used as well-known model cells for *in vitro* studies to investigate the uptake of nanoassemblies and their cytotoxicity effects. Fluorescence microscopy and TEM imaging are performed to characterize the fluorescence properties and the arrangement of the nanoassemblies inside the cells.

***Main results.*** Cell uptake of the nanoassemblies operates within 6-8 h. Cytotoxicity MTT assays reveal that cell viability is not hampered following cell culture with a 15 µmol.L⁻¹ Fe concentration, similar to those used for *in vivo* MRI experiments. Fluorescence microscopy imaging of live cells shows mobile orange bright spots inside the cells, proving no significant aggregation of the internalized nanoparticles. Comparison experiments with fluorescent organic nanospheres prove that the nanoassemblies do not dissociate within the cells since high structural stiffness is brought by the PAA coating. These results are corroborated by 2D TEM imaging of cells showing the intact structure of the nanoassemblies and the circular arrangement of the iron oxide nanoparticles around the organic core.

### B.3.1. Procedures

***Cell incubation.*** The cells were incubated for at least 24 h on an Ibidi 8-well plate at 37.7°C in a 5 % CO₂ atmosphere. 5000 cells were incubated for experiments running over 72 h. MDA cells were grown in RPMI media containing 10 % of fetal bovin serum and 1 % of penicillin and streptomycin. HEK cells were grown in DMEM media (high glucose, with glutamine) containing 10 % of fetal bovin serum and 1 % of penicillin and streptomycin.

***Nanoassembly internalization.*** Internalization was performed by adding to the cell medium a solution of *fluo@mag@PAA* nanoparticles **(1)**dispersed in water (10-20 µL) such that the final concentrations of fluorescent molecules and iron were about 1 × 10⁻⁶ mol.L⁻¹ and 2×10⁻⁵ mol.L⁻¹. The suspension was incubated for 6 h to ensure efficient cell uptake of the nanoassemblies, which could already be observed after 3 h only.

### B.3.2. Cell viability

MTT assay: 5000 cells were grown in the appropriate culture medium (200 µL) for 24 h. They were then incubated for various periods of time with a solution of *fluo@mag@PAA* nanoassemblies **(1)** dispersed in Millipore water. The cell viability was evaluated by using MTT assays. A solution of MTT (20 µL; 5 mg.mL⁻¹) was added to the cells which were incubated for a further 2 h-2 h 30. The supernatant solution was removed and DMSO (200 µL) was added to dissolve the colored oxidized product. Absorbance read-out at 570 nm provides viability of the incubated cells by comparison with reference cells.

The MTT assays reveal almost no cell cytotoxicity for nanoassembly solutions with a 15 µmol.L⁻¹ iron content (cell viability > 95 %) which is the common concentration used for all *in vitro* and *in vivo* experiments. For very large concentrations of iron (300 µmol.L⁻¹), only the HEK cells exhibit significant mortality in agreement with an increase in the oxidative stress caused by the large excess of iron (300-500 µg.mL⁻¹).

### B.3.3. Fluorescence microscopy of internalized nanoassemblies in live cells

Fluorescence microscopy was performed in the confocal mode by means of a LSM working in the inverted mode (Nikon AIR Si, oil-immersion objective Plan Apo, ×60, 1.4, *λ_{exc}* = 488 nm), or in the wide-field mode by means of an inverted microscope (Nikon Eclipse Ti, oil-immersion objective Plan Apo, ×60, 1.4, *λ*_{exc} = 482 nm).

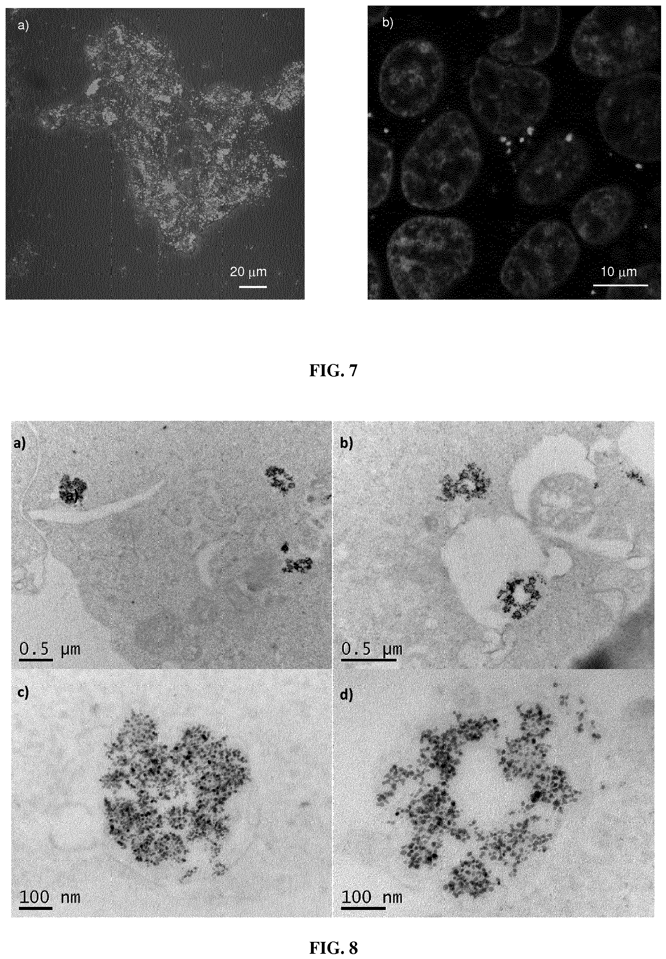

Internalized nanoassemblies **(1)** can clearly be distinguished as spots on **Figure 7a** without undergoing significant aggregation inside the cells. No spots can be distinguished inside the nuclei, indicating that the nanoassemblies stay in the cytoplasm and do not penetrate into the nuclei (**Figure 7b**).

### TEM imaging of internalized nanoassemblies:

- Addition of HEK cells in agar solution at 45-50 °C;
- Fixation for 2 h at 4°C of small pieces of agar gel using a phosphate 0.1 M solution and 3 % glutaraldehyde;
- Washing with phosphate buffer and Millipore water;
- Post-staining using osmium tetraoxide (1 %) in Millipore water for 1 h and repeated washings using Millipore water;
- Dehydratation using an ethanol bath for 1 h with increasing concentration in ethanol (30 %, 50 %, 70 %, 85 %, 95 %, 100 %);
- Dehydratation using a 100% ethanol bath (2 h and overnight at 4 °C);
- Exchange of ethanol with propylene oxide;
- Inclusion in EPON resine;
- Polymerization for 1 day at 55°C and 60 h at 72°C;
- Staining after slicing for 30 min in uranyl acetate and washing using Millipore water.

TEM imaging (**Figure 8**) reveals the embedment of contrasted iron oxide nanoparticles in cell endosomes after cell uptake. Remarkably, the maghemite nanoparticles are organized following a disk-like arrangement recalling the spherical core-shell structure of the nanoassemblies. Given the strong fluorescence detectable in live cells, these TEM images show that the nanoassemblies keep their integrity after internalization with no dissociation of the magnetic shell from the organic core thanks to the cohesive PAA coating.

### B.4. IN VIVO EXPERIMENTS

*In vivo* Magnetic Resonance Imaging was performed on a series of 5 mices. After mice euthanasia, the extracted organs were sliced for fluorescence microscopy imaging using one- and two-photon excitation to minimize tissue auto-fluorescence. In order to gain higher spatial resolution in the imaging of the nanoassemblies, transmission electron microscopy measurements (TEM) were performed on ultra-thin slices of liver (70 nm-thick).

### B.4.1. Magnetic resonance imaging on small rodents

***Procedure.*** T2 weighted MRI experiments were performed on 5 female BALB/C mice (5-week old) by means of a MRI spectrometer BioSpect 4,7 T (Bruker). The echo time TE and the repetition time TR of the sequence (T2* mode) were set at 5 ms and 300 ms respectively. A concentration of 13 µmol Fe /kg mouse was chosen to perform these analyses.

The mouse was anesthetized using isofluorane gas at a concentration allowing for 30 heartbeats per minute. Images of the liver were recorded as references before injecting the nanoassemblies. A suspension of *fluo@mag@PAA* nanoassemblies **(1)** in HBSS buffer (250 µL, 1.3 µmol.mL⁻¹ Fe concentration) was injected in the mouse caudal vein. MRI was performed after 20-30 min. An average spleen ENH of -13% and liver ENH of -49% was obtained.

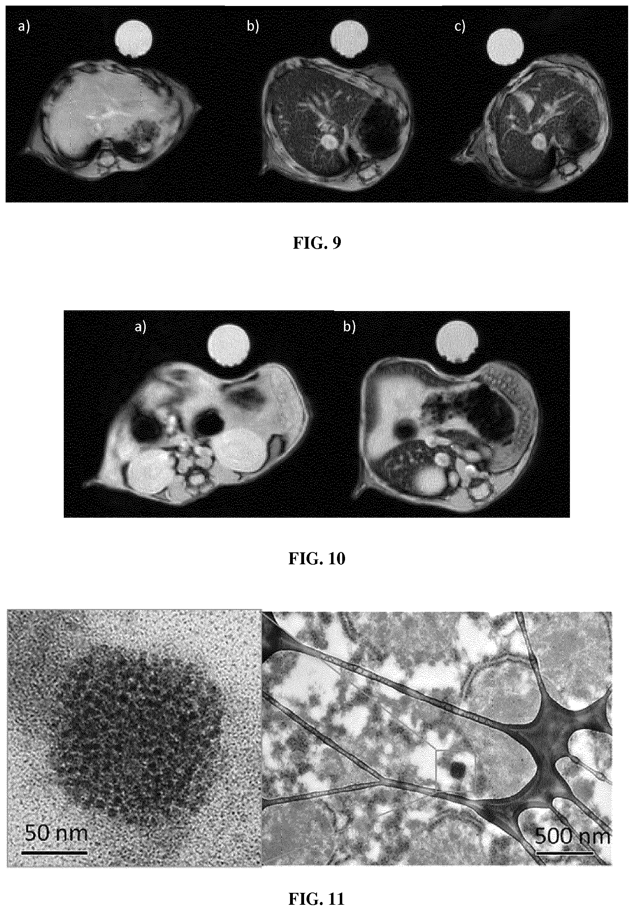

***Results.*** MRI imaging has been performed in order to observe magnetic cooperativity between the iron oxide nanoparticles on the surface of the nanoassembly. As shown on **Figures 9** and **10****,** a negative contrast has been obtained by T2* imaging with iron concentrations (13 µmol Fe /kg) lower than those commonly used with individual iron oxide nanoparticles (average concentration in the literature: 40 - 70 µmol Fe/kg). No contrast evolution was observed after 3 h 50 after injection

T2-weighted MRI experiments are focused on the main organs (spleen and liver) responsible for most of the nanoassembly clearance through the recruitment of macrophages for 150 nm-sized nanoparticles. A clear decrease in the T2* contrast in the liver and spleen can be observed 20-25 min. after the injection. These observations feature efficient uptake of the nanoassemblies. They also demonstrate the efficient role of the *fluo@mag@PAA* nanoassemblies as MRI contrast agents despite the low Fe concentration used. They are to be linked to the high r₂/r₁ ratio of the longitudinal ri and transverse r2 relaxivities (*see Point B.2.4.).*

### B.4.2. Fluorescence microscopy of sliced tissues

*Tissue sectioning procedure.* The mice were sacrificed 24 h after the injection. Five organs (liver, spleen, kidney, lung and heart) were harvested for *ex vivo* analyses in order to assess the biodistribution of the *fluo@mag@PAA* nanoassemblies **(1)**. They were successively immersed in a bath of zinc-containing fixatives (Pharmingen, France) at 4 °C for 48 h, and 4 wt. % paraformaldehyde - 20 wt. % sucrose solution at 4 °C for 24 h. They were eventually frozen in OCT (Tissue-Tek) by using vapors of liquid nitrogen. Zinc-containing fixatives avoid fainting of the fluorescent molecules during the fixing process. The frozen organs were stored at -80°C for 30 days before use. They were cut in 10 µm-thick sections at -20 °C using a cryotome cryostat (Leica Biocut 2030). The sections were immediately deposited on superfrost glass slides, let dry for 2 h before being treated with Prolong (containing or not DAPI as a cell nucleus staining agent) and protected with an upper coverslit to allow for microscopy imaging. The sections were left at 4 °C for at least 72 h before observation.

***Instrumentation.*** Fluorescence microscopy was performed in the confocal mode by means of two kinds of LSM working in the inverted mode:
- *one-photon fluorescence imaging:* Nikon AIR Si equipped with Ar⁺ and He-Ne lasers as excitation sources (use of an oil-immersion objective Plan Apo, ×60, 1.4, *λ_{exc}* = 488 nm) - collaboration IRSUN / Nantes;
- *two-photon fluorescence imaging:* Zeiss LSM 780 equipped with a tunable Ti:*sapphire* femtosecond laser (Mai Tai, 710 - 920 nm) and a 32-channel QUASAR GaAsp detector allowing for multispectral imaging. All images were acquired upon excitation at 830 nm and using two detection channels (blue centered at 450 (±35) nm and red centered at 654 (±104) nm) - collaboration KU Leuven / Belgique.

***Results.*** Fluorescence microscope imaging clearly distinguished *fluo@mag@PAA* nanoassemblies as bright emitting spots in the sliced tissues with no penetration in the cell nuclei. The *fluo@mag@PAA* nanoassemblies efficiently responded to two-photon excitation, allowing for higher localization resolution and NIR investigations in the tissue transparency window. In agreement with the clearance process, the non-functionalized *fluo@mag@PAA* nanoassemblies mostly accumulated in the liver, and in the spleen to a lesser extent. Very few nanoassemblies were found in the kidneys responsible for the clearance of smaller nanoparticles (a few nm-sized) while none of them was found in the lung and heart.

Especially, despite the strong autofluorescence displayed by highly pigmented tissues like liver or spleen, emission of the nanoassemblies was clearly distinguished under one- or two-photon excitations. The nanoassemblies appear as localized small and very bright spots with a fluorescence signal centered at 580 nm, neatly red-shifted compared to the 520 nm-centered autofluorescence. The spot dimensions, close to those of a single nanoassembly, taking into account light scattering, let suggest the absence of nanoassembly aggregation or dissociation *in vivo.* Fluorescence microscope imaging reveals that the nanoassemblies do not enter the hepatocyte cell nuclei. The nanoassemblies exhibit no significant bleaching under normal observation conditions in contrast with the tissue auto-fluorescence, which should enable accurate localization of the nanoassemblies when properly functionalized with targeting agents.

### B.4.3. Transmission electronic microscopy of sliced tissues

### Procedure and reagents

Sodium cacodylate buffer 0.2 mol.L-1, pH = 7.4 (storage over 1 year - 500 mL). Trihydrate sodium cacodylate (21.4 g), dihydrate calcium chloride (25 mg), and hexahydrate magnesium chloride (50 mg) were dissolved in distilled water (450 mL). The pH mixture was adjusted upon adding a 0.5 mol.L-1 HCl solution under magnetic stirring. Distilled water was eventually added to adjust the total volume to 500 mL.

Tissue fixation. The tissues were primarily fixed with glutaraldehyde (2.5 wt. % / vol) for 2 h to preserve their structure. The tissues were subsequently washed with cacodylate buffer for 10 min and postfixed with 2 % tetraoxide osmium for 1 h. They were washed twice in two baths of cacodylate buffer for 5 min each.

Dehydration. Tissue dehydration was performed as follows: 50 % ethanol for 10 min, 70 % ethanol for 10 min, 90 % ethanol for 15 min, 100 % ethanol - 2 changes for 15 min each, propylene oxide - 3 changes for 15 min each.

Resin embedding. Resin infiltration was performed using a succession of a 1:2 mix of resin:propylene oxide for 30 min, a 1:1 mix of resin:propylene oxide for 30 min, and a 2:1 mix of resine:propylene oxide for 30 min. All samples were placed in fresh resin at ambient temperature overnight or at 4°C for 1-2 days. Polymerization was performed in an oven at 60°C for 48 h.

Sample (block) cutting. All blocks were cut as 70 nm-thin sections using a cryo-ultramicrotome (model Reichert Ultracut S) mounted with a diamond knife (Diatome). The sections were placed on the dark side of a holey carbon-coated copper grid (Delta Microscopies).

Post-staining. The grids were floated in a saturated solution of uranyle acetate in 50 % ethanol for 25 min at room temperature and rinsed briefly twice over distilled water. They were left on a drop of lead citrate for 10 min, washed twice with 0.1 mol.L⁻¹ sodium hydroxyde and finally twice with distilled water.

***Results.*** So far, evidence of the *fluo@mag@polymer* nanoassemblies' uptake by liver macrophages was demonstrated by *in vivo* MRI and fluorescence histology fluorescence microscopy experiments. In order to gain higher spatial resolution in the imaging of *fluo@mag@polymer* nanoassemblies, transmission electron microscopy measurements were performed on ultra-thin slices of liver (70 nm-thick). TEM imaging clearly demonstrated the integrity of the administrated nanoassemblies in mice through intraveinous injections since the "raspberry"-like structure of the nanoassemblies is kept integer (**Figure 11**). This important observation proves the high cohesion between the magnetic shell and the fluorescent core after *in vivo* injection, allowing for accurate multimodal cross diagnostics.

### PART C - FUNCTIONALIZED NANOASSEMBLIES

### C.1. (reference example) FOLIC ACID FUNCTIONALIZATION

The *fluo@mag* nanoassemblies may be coated with biologically-active molecules like folic acid (FA) speeding-up the cell internalization cell internalization of nanoparticles. Folic acid was chosen for its complexing carboxylic unit toward the external layer of iron oxide nanoparticles.

Complexation of the bimodal *fluo@mag* nanoassemblies with FA could be realized provided that the resulting nanoassemblies *fluo@mag@FA* are stored in the dark at 4°C. Steady-state and time-resolved fluorescence measurements performed on the resulting architecture *fluo@mag@FA* confirmed the tight FA grafting at the surface of the nanoassemblies.

***Synthetic procedure.*** A solution of fluorescent compound *(Ib)* dissolved in THF (50 µL, 0.1 wt. %) was added under vigorous stirring to a solution of maghemite nanoparticles in nitric acid (2.5 mL, 0.006 wt. %, pH = 1.2). After a few seconds, the magnetofluorescent *fluo@mag* were formed. Folic acid (> 5 eq, 1 mg) was added to the mixture, followed by the dropwise addition of ammonium hydroxide (1 mol.L⁻¹) under magnetic stirring until pH = 8 was reached. The resulting translucent solution was allowed to stir for a further 30 min. and dialyzed using a Spectra Por membrane (Standard Grade Regenerated Cellulose; cut-off: 8-10 kDa) against Millipore water (600 mL) over 3-4 h to remove the excess of folic acid. Once the *fluo@mag* nanoparticles started agglomerating, the dialysis was performed in the presence of trisodium citrate salt (50 mmol.L⁻¹) for a few hours. A final dialysis step was performed against Millipore water (600 mL) to remove the excess of citrate ions. The resulting functionalized *fluo@mag@FA* nanoassemblies were stored at 4 °C to avoid decomplexation of the folic acid units over time.

***Photophysical characterizations.*** Complexation of FA on *fluo@mag* nanoparticles could be detected using steady-state and time-resolved fluorescence measurements. Strong emission *fluo@mag* quenching of the blue fluorescent FA was observed after complexation to the *fluo@mag* nanoparticles due to fast electron transfer from the FA excited state to the iron oxide nanoparticles. By contrast, no significant blue emission quenching could be detected when folic acid was simply mixed with a suspension of iron oxide γ-Fe₂O₃ due to partial complexation only. These results thus demonstrated the cooperative complexing behaviors of ensembles of iron oxide nanoparticles with regard to isolated maghemite nanoparticles. Compared to the tight PAA coating on *fluo@mag* nanoparticles, FA decomplexation however occurred over time. Detection of a strong blue signal thus features uncomplexed FA and has been harnessed to show the release of folic acid in live cells after incubation for 72 h. These results open the possibility to use the *fluo@mag* and *fluo@mag@PAA* nanoassemblies as drug cargos when properly functionalized.

***Fluorescence microscopy imaging of live cells.*** HEK cells were incubated with folic acid-coated *fluo@mag* nanoassemblies (*fluo@mag@FA*) and observed by wide-field fluorescence microscopy. The *fluo@mag@FA* were efficiently internalized in HEK cells. They provided blue emission after 72 h of incubation as a result of the decomplexation of folic acid whereas no such operates after 20 h only. Green emission instead of an orange one was related to the disassembling process of *fluo@mag* nanoassemblies since there was no PAA coating, known to ensure tight cohesion.

### C.2. AMINOCOUMARIN FUNCTIONALIZATION

The *fluo@mag@PAA* nanoassemblies **(1)** could be covalently grafted with biologically-active molecules like aminocoumarine, serving as antiobiotics. The aminocoumarine displays an amino function, amenable to covalently bind the *fluo@mag@PAA* surface through amide bond formation.

The surface of nanoassemblies **(1)** was covalently linked to fluorescent probe 7-amino-4-methylcoumarin (AC) through amide bond formation. Steady-state and time-resolved fluorescence measurements performed on the resulting architecture *fluo@mag@PAA-AC* allowed to confirm the tight AC grafting at the surface of the nanoassemblies and showed the superiority of *fluo@mag@PAA* over *fluo@mag* in terms of surface binding stability of biologically active molecules. Such a functionalization represents the prelude of further grafting of more complex active biomolecules.

***Synthetic procedure.*** Dilute nitric acid (pH = 1.2) was added to a solution of *fluo@mag@PAA* assemblies, dispersed in Millipore water (0.7 µmole Fe, <1 µmole PAA, 2.5 mL) until pH = 4 was reached. N-(3-dimethylaminopropyl)-N'-ethylcarbodiimide hydrochloride (10 eq, 1.4 mg, 10 µmol) and N-hydroxysuccinimide (10 eq, 1.1 mg, 10 µmol) were added to the mixture under vigorous stirring using a vortex. The nanoassemblies were stirred for an extra 2 h before adding ammonium hydroxide (1 mol.L⁻¹) until pH = 9 was reached. Then, 7-amino-4-methylcoumarin (40 eq, 7.0 mg, 40 µmol) was added to the nanoparticle dispersion and the resulting solution was stirred for a further 16 h. The resulting translucent solution was dialyzed using a Spectra Por membrane (Standard Grade Regenerated Cellulose; cut-off: 8-10 kDa) against Millipore water (600 mL) over 72 h until no more blue fluorescent emission was observed in the dialysis bath. The functionalized *fluo@mag@PAA-AC* nanoassemblies were stored at 4 °C.

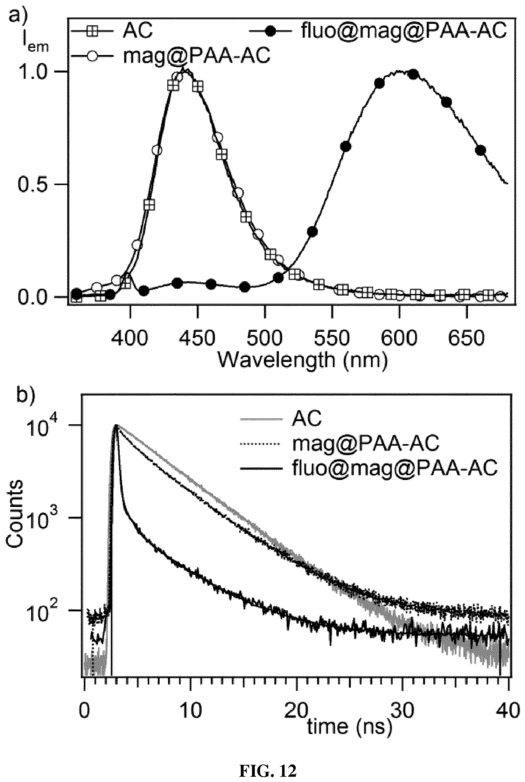

***Photophysical characterizations.*** Steady-state and time-resolved fluorescence experiments were also performed to characterize the surface grafting of 7-amino-4-methylcoumarin, displaying a fluorescence signal at 441 nm (**Figure 12**). Strong emission quenching was detected (decrease in the fluorescence signal and fluorescence decay) for the AC immobilized on the surface of *fluo@mag@PAA* nanoassemblies, compared to water solutions of AC only. This quenching effect was ascribed to efficient energy transfer from the AC excited state to the fluorescent organic core due to spectral overlap between the AC emission spectrum and the fluorescent core's absorption spectrum. The fast component observed in the fluorescence decay for *fluo@mag@PAA-AC* stems from the close proximity of AC toward the iron oxide nanoparticles and consequently to the covalent binding of AC. (**Figure 12b**). No such quenching effect was indeed observed when AC was immobilized on the surface of mag@PAA nanoparticles dispersed in water. This experiment definitely shows that covalent attachment of AC to the *fluo@mag@PAA* nanoassemblies has been achieved since the distance between an energy donor and an energy acceptor must be shorter than 10 nm for efficient energy transfer. Finally no leakage of the covalently linked AC was detected over a period of two weeks. This experiment represents a first step toward the bioconjugation of biologically-active objects proteins for targeted bioimaging and drug delivery.

### C.3. MALEIMIDE FUNCTIONALIZATION

Bioconjugation of *fluo@mag@polymer* requires the introduction of reactive groups known to react efficiently and specifically with biological entities. Maleimide derivatives are especially interesting due to their specific reactivity with thiol groups, present in a minor amount, compared to amino groups, in proteins and antibodies. The introduction of maleimide derivatives proceeded through peptide coupling providing covalent amide linkage between the polymer shell and the maleimide units. The grafting of maleimide was evidenced through fluorescence measurements as the resulting emission spectra underwent a slight hypsochromic shift (Δ*λ*ₘₐₓ ≈ -10 nm) of their emission maxima compared to those before coupling.

***Maleimide derivatives.*** The synthesis of the maleimide derivatives is based on Richter et al (Chem. Eur. J. 2012, 18, 16708-16715). The following maleimide derivatives were coupled to fluo@mag@polymer nanoassemblies **(1)**, 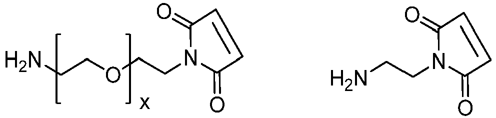 wherein x ranges from 1 to 10, preferably from 3 to 5.

***Maleimide-functionalized ananoassemblies.*** Dilute nitric acid (pH = 1.2) was added to a solution of *fluo@mag@polymer* assemblies, dispersed in Millipore water (4 µmole Fe, 10 mL) until pH = 4 was reached. N-(3-dimethylaminopropyl)-N'-ethylcarbodiimide hydrochloride (4.2 mg, 40 µmol, 10 eq.) and N-hydroxysuccinimide (3.6 mg, 40 µmol, 10 eq.) were added to the mixture under vigorous stirring using a vortex. The nanoassemblies were stirred for an extra 1 h before adding ammonium hydroxide (1 mol.L⁻¹) until pH = 9 was reached.

Then, a trifluoroacetic maleimide salt derivative, as presented herein above, (13.5 mg, 40 µmol, 40 eq.), was added to the nanoparticle dispersion, and the resulting solution was stirred for a further 16 h. The resulting translucent solution was dialyzed using a SpectraPor membrane (Standard Grade Regenerated Cellulose; cut-off: 8-10 kDa) against Millipore water (600 mL) over 24 h. The functionalized nanoassemblies were stored at 4°C.

In order to avoid non-specific interactions, a mixture of maleimide derivatives and commercially available aminoPEG (from 10 to 3000 Da, preferably around 1500 Da) (1/1) could be coupled in the last step of the above reaction.

### C.4 BIOTIN FUNCTIONALIZATION

The fluo@mag@polymer were functionalized by covalent conjugation with biotin that is a very common ligand used in biology to complex various entities of biological interest coupled to streptavidin. As biotin and streptavidin form an affinity complex with a high stability constant (Kd = 10⁸), the following results demonstrate that fluo@mag@polymer functionalized with biotin can readily be used to recognize streptavidin-conjugated biological entities.

***Procedure.*** Dilute nitric acid (pH = 1.2) was added to a solution of *fluo@mag@polymer* assemblies **(1)**, dispersed in Millipore water (2 µmole Fe, 5 mL) until pH = 4 was reached. N-(3-dimethylaminopropyl)-N'-ethylcarbodiimide hydrochloride (2.8 mg, 20 µmol, 10 eq.) and N-hydroxysuccinimide (2.4 mg, 20 µmol, 10 eq.) were added to the mixture under vigorous stirring using a vortex. The nanoassemblies were stirred for an extra 1 h before adding ammonium hydroxide (1 mol.L⁻¹) until pH = 9 was reached. Then, biotin-X-cadaverin (1 mg, 2 µmol, 1 eq.) was added to the nanoparticle dispersion and the resulting solution was stirred for a further 16 h. The resulting translucent solution was dialyzed using a SpectraPor membrane (Standard Grade Regenerated Cellulose; cut-off: 8-10 kDa) against Millipore water (600 mL) over 24 h. The biotin-functionalized nanoassemblies were stored at 4°C.

In order to avoid non-specific interactions, a mixture of biotin-X-cadaverine and commercially available aminoPEG (from 10 to 3000 Da, preferably around 1500 Da) (1/1) can be coupled in the last step of above reaction.

***Detection, extraction and quantification.*** The possibility of bio-functionalized nanoassemblies to serve as sensing agents has been tested by performing the well-known biotin/streptavidin association. The biotin-functionalized nanoassemblies were lyophilized and then redispersed in appropriate buffer and streptavidin conjugated to Alexa Fluor® 488 was added. After stirring for 1 h at room temperature, dialysis was performed to remove the excess of fluorescent streptavidin.

Fluorescence emission measurements of the dialyzed solution displayed a narrow emission signal around 525 nm typical of Alexa Fluor® 488 attached to streptavidin, along with the broad red fluorescence characteristic of the core (> 600 nm). This signal allowed to unambiguously prove the association of fluorescent streptavidin with the biotin attached to the nanoassembly. Assuming that the emission of fluorophore Alexa Fluor® 488 did not change upon biotin-streptavidin association, an amount of about 100 biotins could be inferred from measuring the fluorescence signal intensity coming from Alexa Fluor® 488.

## Claims

1. A nanoassembly system comprising
- a nanoassembly comprising:
∘ an organic fluorescent inner core comprising fluorescent organic molecules, and
∘ magnetic nanoparticles contacting the surface of said fluorescent inner core; and
- at least one polymer adsorbed at the surface of the nanoassembly; provided that the nanoassembly system does not comprise silica, functionalized silica, doped silica, grafted silica or a combination thereof,
wherein the fluorescent organic molecules are compounds of Formula (I), 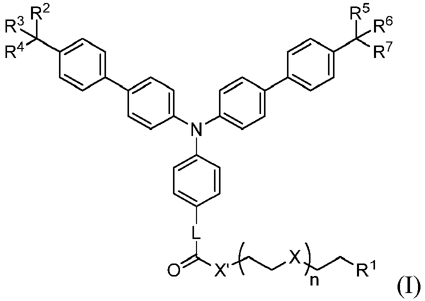 wherein:
**L** represents a spacer selected from (i), (ii) and (iii) 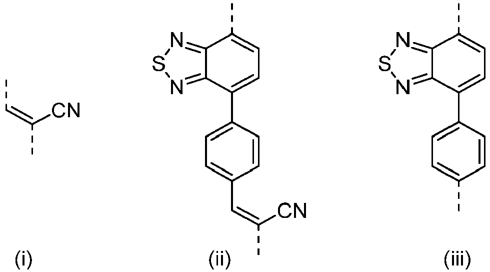
**X** represents O, CH₂ or NR⁸, wherein R⁸ represents H, alkyl or alkene;
**X'** represents O or NR⁹, wherein R⁹ represents H, alkyl or alkene;
**n** represents an integer selected from 1, 2, 3 and 4;
**R¹** represents -CO₂H or -P(O)(OH)₂;
**R², R³, R⁴, R⁵, R⁶** and **R⁷** represent each independently an optionally functionalized group selected from alkyl and ester or poly(ethylene glycol), preferably **R², R³, R⁴, R⁵, R⁶** and **R⁷** represent each a methyl group, and
wherein the magnetic nanoparticles are selected from the group consisting of γ-Fe₂O₃, Fe₃O₄, CoFe₂O₄, MnFe₂O₄, CuFe₂O₄ and NiFe₂O₄.

2. The nanoassembly system according to claim **1,** wherein the magnetic nanoparticles are superparamagnetic nanoparticles selected from the group consisting of γ-Fe₂O₃ and Fe₃O₄.

3. The nanoassembly system according to any one of claims **1** to **2,** having a hydrodynamic diameter ranging from 20 to 700 nm.

4. The nanoassembly system according to any one of claims **1** to **3,** wherein the fluorescent inner core comprises a number of organic fluorescent molecule ranging from 1×10⁴ to 1×10⁷.

5. The nanoassembly system according to any one of claims **1** to **4,** comprising a number of magnetic nanoparticles ranging from 1×10² to 1×10⁶.

6. The nanoassembly system according to any one of claims **1** to **5,** wherein the polymer is an ionic polymer, preferably a polyelectrolyte.

7. The nanoassembly system according to any one of claims **1** to **6,** wherein the polymer is of Formula (II), 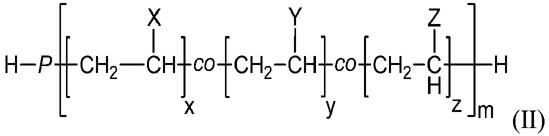 wherein,
**m** represents a positive integer ranging from 20 to 150;
**x, y** and **z** represent each independently a percentage of **m,** ranging from 0% to 100% of m, wherein x+y+z is equal to 100% of m;
**X** represents -COOH, alkyl, aryl, -CO-alkyl, poly(ethylene glycol), -poly(ethylene glycol)-OMe; **Y** represents -(C=O)-O-L¹-R⁸, -(C=O)-S-L¹-R⁸, - (C=O)-NH(-L¹-R⁸) or -(C=S)-NH(-L¹-R⁸) wherein
**L¹** represents a spacer selected from alkyl, alkene, aryl, arylalkyl, poly(ethylene glycol) or poly(propylene glycol) linking groups having 1 to 150 chain atoms, wherein the linking group can be optionally interrupted or terminated by one or more -O-, -S-, -NR⁹-, -CO-, -NHCO-, -CONH- or a combination thereof, wherein R⁹ is H or alkyl;
**R⁸** represents a reactive group selected from N₃, amino, alkylamino, COOH, amide, maleimide, alkene, alkyne, SH, OH, ester, activated ester, activated carboxylic acid, halo, nitro, nitrile, isonitriles, acrylamide, aldehyde, ketone, acetal, ketal, anhydride, glutaric anhydride, succinic anhydride, maleic anhydride, thiocyanate, isothiocyanate, isocyanate, hydrazide, hydrazine, hydrazone, ether, oxide, cyanate, diazo, diazonium, sulfide, disulfide, sulfoxide, sulfone, sulfonic acid, sulfinic acid, sulfate, sulfenic acid, amidine, imide, imidate, nitrone, hydroxylamine, oxime, hydroxamic acid, thiohydroxamic acid, allene, ortho ester, sulfite, enamine, ynamine, urea, pseudourea, semicarbazide, carbodiimide, carbamate, imine, acetylene, olefin, polyene, alkylacrylate, oxetane, ammonium, oxonium, phosphonium, sulfonium, positively charged or neutral metal complex;
**Z** represents -(C=O)-O-L²-R¹⁰, -(C=O)-S-L²-R¹⁰, -(C=O)-NH(-L²-R¹⁰) or -(C=S)-NH(-L²-R¹⁰) wherein
**L²** represents a single bond or a spacer selected from alkyl, alkene, aryl, arylalkyl, poly(ethylene glycol) or poly(propylene glycol) linking groups having 1 to 150 chain atoms, wherein the linking group can be optionally interrupted or terminated by one or more -O-, -S-, -NR⁹-, -CO-, -NHCO-, -CONH- or a combination thereof, wherein R⁹ is H or alkyl; optionally additionally comprising a residue of a reactive group through which L² is bonded to R¹⁰;
**R¹⁰** represents a bioactive group selected from amino acid, peptide, protein, antibody, affinity protein, enzyme, polysaccharide, dextran, benzylguanine, lipid, lipid assembly, fatty acid, nucleoside, nucleotide, oligonucleotide, hapten, aptamer, ligand, antibiotic, substrate, biotin, avidin, streptavidin, synthetic polymer, poly(ethylene glycol), poly(propylene glycol), polymeric microparticle, nanoparticle, fluorophore, chromophore, radioisotope, macrocyclic complexes of radioisotope, and combinations thereof.

8. The nanoassembly system according to any one of claims **1** to **7,** wherein the polymer is poly(acrylic acid).

9. A process for manufacturing nanoassembly systems according to any one of claims **1** to **8,** comprising injecting a solution of fluorescent organic molecules into a dispersion of magnetic nanoparticles under stirring, and a subsequent step of addition of the polymer.

10. The process according to claim **9,** further comprising a subsequent step of functionalization of the polymer by a bioactive group.

11. Pharmaceutical composition comprising nanoassembly systems according to any one of claims **1** to **8,** in combination with at least one pharmaceutically acceptable vehicle.

12. Medicament comprising nanoassembly systems according to any one of claims **1** to **8.**

13. Use of nanoassembly systems according to any one of claims **1** to **8,** to extract and detect molecules and/or biological targets from physiological medium.

14. Kit comprising nanoassembly systems according to any one of claims **1** to **8.**

## Patentansprüche

1. Nanoanordnungssystem, umfassend
- eine Nanoanordnung, welche umfasst:
∘ einen organischen fluoreszierenden inneren Kern, der fluoreszierende organische Moleküle umfasst, und
∘ magnetische Nanopartikel, die die Oberfläche des fluoreszierenden inneren Kerns berühren; und
- mindestens ein Polymer, das an der Oberfläche der Nanoanordnung adsorbiert ist; vorausgesetzt, dass das Nanoanordnungssystem kein Siliciumdioxid, funktionalisiertes Siliciumdioxid, dotiertes Siliciumdioxid, gepfropftes Siliciumdioxid oder eine Kombination davon umfasst,
wobei es sich bei den fluoreszierenden organischen Molekülen um Verbindungen der Formel (I) handelt 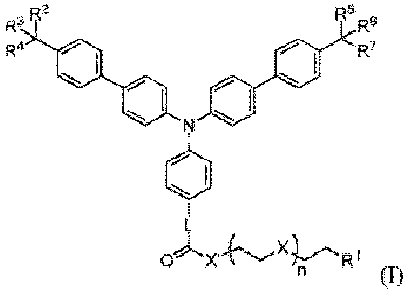 wobei:
**L** für einen Spacer steht, ausgewählt aus (i), (ii) und (iii) 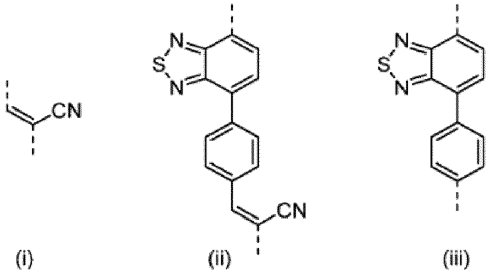
**X** für O, CH₂ oder NR⁸ steht, wobei R⁸ für H, Alkyl oder Alken steht;
**X'** für O oder NR⁹ steht, wobei R⁹ für H, Alkyl oder Alken steht;
**n** für eine ganze Zahl steht, ausgewählt aus 1, 2, 3 und 4;
**R¹** für -CO₂H oder -P(O)(OH)₂ steht;
**R², R³, R⁴, R⁵, R⁶** und **R⁷** jeweils unabhängig für eine gegebenenfalls funktionalisierte Gruppe stehen, ausgewählt aus Alkyl und Ester oder Poly(ethylenglycol), vorzugsweise **R², R³, R⁴, R⁵, R⁶** und **R⁷** jeweils für eine Methylgruppe stehen, und
wobei die magnetischen Nanopartikel ausgewählt sind aus der Gruppe bestehend aus γ-Fe₂O₃, Fe₃O₄, CoFe₂O₄, MnFe₂O₄, CuFe₂O₄ und NiFe₂O₄.

2. Nanoanordnungssystem nach Anspruch **1,** wobei es sich bei den magnetischen Nanopartikeln um superparamagnetische Nanopartikel handelt, ausgewählt aus der Gruppe bestehend aus γ-Fe₂O₃ und Fe₃O₄.

3. Nanoanordnungssystem nach einem der Ansprüche **1** bis **2,** das einen hydrodynamischen Durchmesser im Bereich von 20 bis 700 nm aufweist.

4. Nanoanordnungssystem nach einem der Ansprüche **1** bis **3,** wobei der fluoreszierende innere Kern eine Anzahl von organischen fluoreszierenden Molekülen im Bereich von 1x10⁴ bis 1x10⁷ umfasst.

5. Nanoanordnungssystem nach einem der Ansprüche **1** bis **4,** das eine Anzahl von magnetischen Nanopartikeln im Bereich von 1x10² bis 1x10⁶ umfasst.

6. Nanoanordnungssystem nach einem der Ansprüche **1** bis **5,** wobei es sich bei dem Polymer um ein ionisches Polymer, vorzugsweise einen Polyelektrolyten handelt.

7. Nanoanordnungssystem nach einem der Ansprüche **1** bis **6,** wobei das Polymer der Formel (II) entspricht 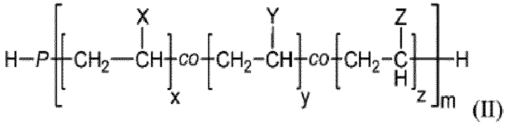 wobei
**m** für eine positive ganze Zahl im Bereich von 20 bis 150 steht;
**x, y** und **z** jeweils unabhängig für einen Prozentsatz von **m** im Bereich von 0 % bis 100 % von m stehen, wobei x+y+x gleich 100 % von m ist;
**X** für -COOH, Alkyl, Aryl, -CO-Alkyl, Poly(ethylenglycol), -Poly(ethylenglycol)-OMe steht; **Y** für -(C=O)-O-L¹-R⁸, -(C=O)-S-L1-R⁸, -(C=O)-NH(-L¹-R⁸) oder -(C=S)-NH(-L¹⁻R⁸) steht, wobei
**L¹** für einen Spacer steht, ausgewählt aus Alkyl-, Alken-, Aryl-, Arylalkyl-, Poly(ethylenglycol)- oder Poly(propylenglycol)-Verbindungsgruppen mit 1 bis 150 Kettenatomen, wobei die Verbindungsgruppe gegebenenfalls durch ein oder mehrere -O-, -S-, -NR⁹-, -CO-, -NHCO-, -CONH- oder eine Kombination davon unterbrochen oder terminiert sein kann, wobei es sich bei R⁹ um H oder Alkyl handelt;
**R⁸** für eine reaktive Gruppe steht, ausgewählt aus N3, Amino, Alkylamino, COOH, Amid, Maleimid, Alken, Alkin, SH, OH, Ester, aktiviertem Ester, aktivierter Carbonsäure, Halogen, Nitro, Nitril, Isonitrilen, Acrylamid, Aldehyd, Keton, Acetal, Ketal, Anhydrid, Glutarsäureanhydrid, Bernsteinsäureanhydrid, Maleinsäureanhydrid, Thiocyanat, Isothiocyanat, Isocyanat, Hydrazid, Hydrazin, Hydrazon, Ether, Oxid, Cyanat, Diazo, Diazonium, Sulfid, Disulfid, Sulfoxid, Sulfon, Sulfonsäure, Sulfinsäure, Sulfat, Sulfensäure, Amidin, Imid, Imidat, Nitron, Hydroxylamin, Oxim, Hydroxamsäure, Thiohydroxamsäure, Allen, Orthoester, Sulfit, Enamin, Ynamin, Harnstoff, Pseudoharnstoff, Semicarbazid, Carbodiimid, Carbamat, Imin, Acetylen, Olefin, Polyen, Alkylacrylat, Oxetan, Ammonium, Oxonium, Phosphonium, Sulfonium, positiv geladenem oder neutralem Metallkomplex;
**Z** für -(C=O)-O-L²-R¹⁰, -(C=O)-S-L²-R¹⁰, -(C=O)-NH(-L²-R¹⁰) oder -(C=S)-NH(-L²-R¹⁰) steht, wobei
**L²** für eine Einfachbindung oder einen Spacer steht, ausgewählt aus Alkyl-, Alken-, Aryl-, Arylalkyl-, Poly(ethylenglycol)- oder Poly(propylenglycol)-Verbindungsgruppen mit 1 bis 150 Kettenatomen, wobei die Verbindungsgruppe gegebenenfalls durch ein oder mehrere -O-, -S-, -NR⁹-, - CO-, -NHCO-, -CONH- oder eine Kombination davon unterbrochen oder terminiert sein kann, wobei es sich bei R⁹ um H oder Alkyl handelt; gegebenenfalls zusätzlich einen Rest einer reaktiven Gruppe umfassend, durch den L² an R¹⁰ gebunden ist;
**R¹⁰** für eine bioaktive Gruppe steht, ausgewählt aus Aminosäure, Peptid, Protein, Antikörper, Affinitätsprotein, Enzym, Polysaccharid, Dextran, Benzylguanin, Lipid, Lipidanordnung, Fettsäure, Nukleosid, Nukleotid, Oligonukleotid, Hapten, Aptamer, Ligand, Antibiotikum, Substrat, Biotin, Avidin, Streptavidin, synthetischem Polymer, Poly(ethylenglykol), Poly(propylenglykol), polymerem Mikropartikel, Nanopartikel, Fluorophor, Chromophor, Radioisotop, makrozyklischen Komplexen von Radioisotop, und Kombinationen davon.

8. Nanoanordnungssystem nach einem der Ansprüche **1** bis **7,** wobei es sich bei dem Polymer um Poly(acrylsäure) handelt.

9. Verfahren zur Herstellung von Nanoanordnungssystemen nach einem der Ansprüche **1** bis **8,** das das Injizieren einer Lösung fluoreszierender organischer Moleküle in eine Dispersion magnetischer Nanopartikel unter Rühren, und einen anschließenden Schritt des Zugebens des Polymers umfasst.

10. Verfahren nach Anspruch **9,** das weiter einen anschließenden Schritt des Funktionalisierens des Polymers durch eine bioaktive Gruppe umfasst.

11. Pharmazeutische Zusammensetzung, die Nanoanordnungssysteme nach einem der Ansprüche **1** bis **8** in Kombination mit mindestens einem pharmazeutisch verträglichen Vehikel umfasst.

12. Medikament, das Nanoanordnungssysteme nach einem der Ansprüche **1** bis **8** umfasst.

13. Verwendung von Nanoanordnungssystemen nach einem der Ansprüche **1** bis **8,** um Moleküle und/oder biologische Ziele aus physiologischem Medium zu extrahieren und nachzuweisen.

14. Set, das Nanoanordnungssysteme nach einem der Ansprüche **1** bis **8** umfasst.

## Revendications

1. Système de nanoassemblage comprenant
- un nanoassemblage comprenant :
∘ un noyau interne fluorescent organique comprenant des molécules organiques fluorescentes, et
∘ des nanoparticules magnétiques en contact avec la surface dudit noyau interne fluorescent ; et
- au moins un polymère adsorbé à la surface du nanoassemblage ;
à condition que le système de nanoassemblage ne comprenne pas de silice, de silice fonctionnalisée, de silice dopée, de silice greffée ou une combinaison de celles-ci,
dans lequel les molécules organiques fluorescentes sont des composés de Formule (I), 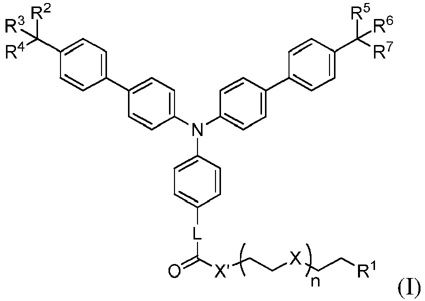 dans laquelle :
**L** représente un espaceur choisi parmi (i), (ii) et (iii) 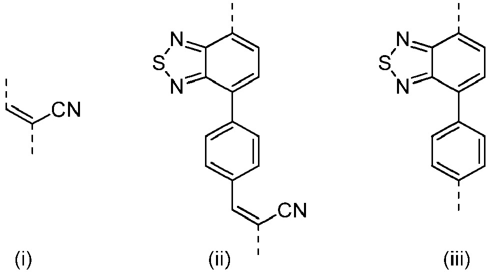
**X** représente O, CH₂ ou NR⁸, dans lequel R⁸ représente H, alkyle ou alcène ;
**X'** représente O ou NR⁹, dans lequel R⁹ représente H, alkyle ou alcène ;
**n** représente un nombre entier choisi parmi 1, 2, 3 et 4 ;
**R¹** représente -CO₂H ou -P(O)(OH)₂;
**R², R³, R⁴, R⁵, R⁶** et **R⁷** représentent chacun indépendamment un groupe éventuellement fonctionnalisé choisi parmi alkyle et ester ou poly(éthylène glycol), de préférence **R², R³**, **R⁴, R⁵, R⁶** et **R⁷** représentent chacun un groupe méthyle, et
dans lequel les nanoparticules magnétiques sont choisies dans le groupe constitué par γ-Fe₂O₃, Fe₃O₄, CoFe₂O₄, MnFe₂O₄, CuFe₂O₄ et NiFe₂O₄.

2. Système de nanoassemblage selon la revendication **1,** dans lequel les nanoparticules magnétiques sont des nanoparticules superparamagnétiques choisies dans le groupe constitué par γ-Fe₂O₃ et Fe₃O₄.

3. Système de nanoassemblage selon l'une quelconque des revendications **1** à **2,** ayant un diamètre hydrodynamique allant de 20 à 700 nm.

4. Système de nanoassemblage selon l'une quelconque des revendications **1** à **3,** dans lequel le noyau interne fluorescent comprend un nombre de molécules fluorescentes organiques allant de 1×10⁴ à 1×10⁷.

5. Système de nanoassemblage selon l'une quelconque des revendications **1** à **4,** comprenant un nombre de nanoparticules magnétiques allant de 1×10² à 1×10⁶.

6. Système de nanoassemblage selon l'une quelconque des revendications **1** à **5,** dans lequel le polymère est un polymère ionique, de préférence un polyélectrolyte.

7. Système de nanoassemblage selon l'une quelconque des revendications **1** à **6,** dans lequel le polymère est de Formule (II), 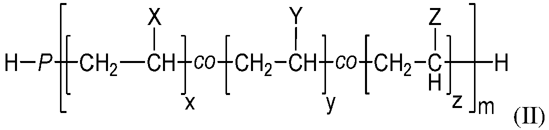 dans laquelle,
**m** représente un nombre entier positif allant de 20 à 150 ;
**x, y** et **z** représentent chacun indépendamment un pourcentage de **m,** allant de 0% à 100% de m, où x+y+z est égal à 100 % de m ;
**X** représente -COOH, alkyle, aryle, -CO-alkyle, poly(éthylène glycol), -poly(éthylène glycol)-Ome ; **Y** représente -(C=O)-O-L¹-R⁸, -(C=O)-S-L¹-R⁸, - (C=O)-NH(-L¹-R⁸) ou -(C=S)-NH(-L¹-R⁸) dans lequel
**L¹** représente un espaceur choisi parmi les groupes de liaison alkyle, alcène, aryle, arylalkyle, poly(éthylène glycol) ou poly(propylène glycol) ayant 1 à 150 atomes de chaîne, dans lequel le groupe de liaison peut éventuellement être interrompu ou terminé par un ou plusieurs -O-, -S-, -NR⁹-, -CO-, -NHCO-, - CONH- ou une combinaison de ceux-ci, où R⁹ est H ou alkyle ;
**R⁸** représente un groupe réactif choisi parmi N₃, amino, alkylamino, COOH, amide, maléimide, alcène, alcyne, SH, OH, ester, ester activé, acide carboxylique activé, halo, nitro, nitrile, isonitriles, acrylamide, aldéhyde, cétone, acétal, cétal, anhydride, anhydride glutarique, anhydride succinique, anhydride maléique, thiocyanate, isothiocyanate, isocyanate, hydrazide, hydrazine, hydrazone, éther, oxyde, cyanate, diazo, diazonium, sulfure, disulfure, sulfoxyde, sulfone, acide sulfonique, acide sulfinique, sulfate, acide sulfénique, amidine, imide, imidate, nitrone, hydroxylamine, oxime, acide hydroxamique, acide thiohydroxamique, allène, ortho ester, sulfite, énamine, ynamine, urée, pseudourée, semicarbazide, carbodiimide, carbamate, imine, acétylène, oléfine, polyène, alkylacrylate, oxétane, ammonium, oxonium, phosphonium, sulfonium, complexe métallique chargé positivement ou neutre ;
**Z** représente -(C=O)-O-L²-R¹⁰, -(C=O)-S-L²-R¹⁰, -(C=O)-NH(-L²-R¹⁰) ou -(C=S)-NH(-L²-R¹⁰) dans lequel
**L²** représente une liaison simple ou un espaceur choisi parmi les groupes de liaison alkyle, alcène, aryle, arylalkyle, poly(éthylène glycol) ou poly(propylène glycol) ayant de 1 à 150 atomes de chaîne, dans lequel le groupe de liaison peut être facultativement interrompu ou terminé par un ou plusieurs -O-, -S-, -NR⁹-, -CO-, -NHCO-, -CONH- ou une combinaison de ceux-ci, où R⁹ est H ou alkyle ; facultativement comprenant en outre un résidu d'un groupe réactif par lequel L² est lié à R¹⁰ ;
**R¹⁰** représente un groupe bioactif choisi parmi acide aminé, peptide, protéine, anticorps, protéine d'affinité, enzyme, polysaccharide, dextrane, benzylguanine, lipide, assemblage lipidique, acide gras, nucléoside, nucléotide, oligonucléotide, haptène, aptamère, ligand, antibiotique, substrat, biotine, avidine, streptavidine, polymère synthetique, poly(éthylène glycol), poly(propylène glycol), microparticule polymerique, nanoparticule, fluorophore, chromophore, radio-isotope, complexes macrocycliques de radio-isotope, et des combinaisons de ceux-ci.

8. Système de nanoassemblage selon l'une quelconque des revendications **1** à **7,** dans lequel le polymère est le poly(acide acrylique).

9. Procédé de fabrication de systèmes de nanoassemblage selon l'une quelconque des revendications **1** à **8,** comprenant l'injection d'une solution de molécules organiques fluorescentes dans une dispersion de nanoparticules magnétiques sous agitation, et une étape ultérieure d'addition du polymère.

10. Procédé selon la revendication **9,** comprenant en outre une étape ultérieure de fonctionnalisation du polymère par un groupe bioactif.

11. Composition pharmaceutique comprenant des systèmes de nanoassemblage selon l'une quelconque des revendications **1** à **8,** en combinaison avec au moins un véhicule pharmaceutiquement acceptable.

12. Médicament comprenant des systèmes de nanoassemblage selon l'une quelconque des revendications **1** à **8.**

13. Utilisation de systèmes de nanoassemblage selon l'une quelconque des revendications **1** à **8,** pour extraire et détecter des molécules et/ou des cibles biologiques à partir d'un milieu physiologique.

14. Kit comprenant des systèmes de nanoassemblage selon l'une quelconque des revendications **1** to **8.**
